# EUROPEAN PATENT SPECIFICATION

(11) **EP 4 096 628 B1**
(45) Date of publication and mention of the grant of the patent: **24.01.2024**
(21) Application number: 21705878.3
(22) Date of filing: 29.01.2021
(51) Int. Cl.: A61K 31/502, A61P 11/00, A61K 9/00

(54) **USE OF 5-AMINO-2,3-DIHYDRO-1,4-PHTHALAZINEDIONE IN THE INHALATORY TREATMENT OF INFLAMMATORY PULMONARY DISEASES**
VERWENDUNG VON 5-AMINO-2,3-DIHYDRO-1,4-PHTHALAZINDION ZUR INHALATIVEN BEHANDLUNG VON ENTZÜNDLICHEN LUNGENERKRANKUNGEN
UTILISATION DE 5-AMINO-2,3-DIHYDRO-1,4-PHTHALAZINEDIONE DANS LE TRAITEMENT PAR INHALATION DE MALADIES PULMONAIRES INFLAMMATOIRES

(30) Priority: 31.01.2020 EP 20000051
(43) Date of publication of application: 07.12.2022
(73) Proprietor: MetrioPharm AG, 8021 Zürich (CH)
(72) Inventor: BRYSCH, Wolfgang, 13505 Berlin (DE); KAISER, Astrid, 12305 Berlin (DE); SCHULZ, Petra, 13158 Berlin (DE); SCHUMANN, Sara, 14554 Seddiner See (DE); von WEGERER, Jörg, 13597 Berlin (DE)
(74) Representative: Gruber, Daniel
(86) International application number: PCT/EP2021/000009
(87) International publication number: WO 2021/151619

(56) References cited:
- EP-A1- 3 416 951
- WO-A1-2018/082814
- RU-C2- 2 266 119
- YU-MEI KUO ET AL: "Characterization of Vibrating Mesh Aerosol Generators", AEROSOL AND AIR QUALITY RESEARCH, vol. 19, no. 8, 1 January 2019 (2019-01-01), pages 1678-1687, XP055740320, ISSN: 1680-8584, DOI: 10.4209/aaqr.2018.11.0436

## Description

The present invention relates to the use of 5-amino-2,3-dihydro-1,4-phthalazinedione or its pharmaceutically acceptable salts in the inhalatory treatment of inflammatory pulmonary diseases as defined by the claims. The invention in particular relates to the use of 5-amino-2,3-dihydro-1,4-phthalazinedione sodium salt for said purposes. Advantageous features of an aerosol containing 5-amino-2,3-dihydro-1,4-phthalazinedione or its pharmaceutically acceptable salts and a method for producing said aerosol are disclosed. The present invention further relates to a kit for inhalatory treatment of inflammatory pulmonary diseases as defined by the claims.

### BACKGROUND OF THE INVENTION

Pulmonary diseases affect the lower airways of the respiratory system, in particular the lungs. This term encompasses pathological conditions that impair the gas exchange in the lungs or bronchi in mammals. In general, they are differentiated into obstructive and restrictive pulmonary diseases. Obstructive pulmonary diseases are characterized by airway obstruction. This limits the amount of air that is able to enter the alveoli because of constriction of the bronchial tree, due to inflammation. Restrictive pulmonary diseases are characterized by a loss of lung compliance, causing incomplete lung expansion and increased lung stiffness.

They can be also categorized as airway diseases, lung tissue diseases, lung infectious diseases and lung proliferative diseases. Airway diseases affect the tubes that carry oxygen and other gases into and out of the lungs. They usually cause a narrowing or blockage of the airways. Typical airway diseases include asthma, chronic obstructive pulmonary disease (COPD) and bronchiectasis. Lung tissue diseases affect the structure of the lung tissue. Scarring or inflammation of the tissue makes the lungs unable to expand fully. This complicates the gas exchange. As a result, these patients cannot breathe deeply. Pulmonary fibrosis and sarcoidosis are typical examples thereof. Lung infectious diseases refer to disorders caused by an infection of the lower airways, e.g. pneumonia. Lung proliferative diseases include all tumors or neoplasms of the lower airways.

Most of the airway diseases are caused by an underlying inflammation or at least include an inflammatory component. Lung tissue diseases have often also an inflammatory component unless they are caused by direct physical impairment of the respiratory tract. Infectious and proliferative diseases of the lungs may also have an inflammatory component, often secondary to the infection or the underlying malignancy.

Thus, these inflammatory pulmonary diseases have in common that they could be pharmacologically treated by anti-inflammatory drugs. However, therapeutic efficacy is often hampered by insufficient availability, respectively efficacy of the drug at the site of inflammation in the lung. Systemic administration, e.g. orally or parenterally, yields often no or only an insufficient therapeutic success.

An alternative administration route is inhalation. Metered-dose inhalers (MDI) are widely in use, e.g. in the treatment of asthma. They use to have a container, respectively canister for the pharmaceutical formulation, a metering valve, for metering the dispensed quantity and a mouthpiece for inhaling. The pharmaceutical formulation consists of the drug, a liquefied gas propellant such as hydrofluoroalkanes and optionally pharmaceutically acceptable excipients.

A specific group of MDIs are dry powder inhalers (DPI). They deliver the drug to the lungs in the form of a dry powder. Most DPIs rely on the force of patient inhalation to entrain powder from the device and subsequently break-up the powder into particles that are small enough to reach the lungs. For this reason, insufficient patient inhalation flow rates may lead to reduced dose delivery and incomplete disaggregation of the powder, leading to unsatisfactory device performance. Thus, most DPIs need a minimum inspiratory effort for proper use. Therefore, their use is limited to older children and adults.

While disorders affecting the bronchi or upper parts of the lower airways can be addressed this way, e.g. by asthma sprays, disorders affecting the alveoli where the gas exchange takes place can be only insufficiently treated because of ineffective inhalatory administration, e.g. COPD. The administered drug particles are not able to reach the bottom of the lungs by way of inhalation, at least not in a therapeutically effective amount.

Nebulizers use to administer the active principle in the form of a mist inhaled into the lungs. Physically, this mist is an aerosol. It is generated in the nebulizer by breaking up solutions and suspensions into small aerosol droplets that can be directly inhaled from the mouthpiece of the device. In conventional nebulizers the aerosol can be generated by mechanical force, e.g. spring force in soft mist nebulizers, or electrical force. In jet nebulizers a compressor brings oxygen or compressed air to flow at high velocity through the aqueous solution with the active principle, this way generating an aerosol. A variant are pressurized metered-dose inhalers (pMDls). Ultrasonic wave nebulizers use an electronic oscillator that at high frequency causes vibration of a piezoelectric element for generating ultrasonic waves in the liquid reservoir with the active principle.

The most promising technology are vibrating mesh nebulizers. They use a mesh, respectively a polymer membrane having a very large number of laser-drilled holes. This membrane is placed between the liquid reservoir and the aerosol chamber. A piezoelectric element placed on the membrane induces high frequency vibrations of the membrane, leading to droplet formation in the aqueous solution and pressuring these droplets through the holes of the membrane into the aerosol chamber. With this technique very small droplet sizes can be generated. Moreover, a significantly shorter inhalation time for the patient can thus be achieved, a feature which drastically increases patient compliance. Only these mesh nebulizers are regarded to be able to generate liquid droplets with the active principle in the desired size range and bring them in a therapeutically effective amount into the patient's alveoli in a reasonable time.

An overview of the characterization of vibrating mesh aerosol generators is given in Kuo et al. (2019) Aerosol and Air Quality Research 19: 1678-1687. Neither the substance of the invention nor the specific ranges of aerosol parameters of the substance of the invention are disclosed therein.

However, not all agents that might be effective in the pharmaceutic treatment of inflammatory pulmonary diseases are suitable for the mesh nebulization technology. For example, some agents are nearly insoluble in water, or their intrinsic physicochemical molecule properties don't allow for the generation of an aerosol in the desired particle size range. Therefore, many nebulized anti-inflammatory agents met only mixed success in the treatment of inflammatory pulmonary diseases.

Thus, there is a medical need to find a pharmaceutical agent that shows a high efficacy in the treatment of inflammatory pulmonary diseases and at the same time allows for the generation of an aerosol in the desired particle size range in order to be able to reach the alveoli of a patient in need thereof.

Surprisingly, this task could be solved by the nebulization of 5-amino-2,3-dihydro-1,4-phthalazinedione or one of its pharmaceutically acceptable salts or solvates, hydrates, crystalline polymorphs, tautomers or isotopically enriched forms thereof.

### DETAILED DESCRIPTION OF THE INVENTION

5-amino-2,3-dihydro-1,4-phthalazinedione belongs to the pharmaceutical class of the phthalazinediones. Compounds of this class are known for their beneficial anti-inflammatory action. 5-amino-2,3-dihydro-1,4-phthalazinedione is also known under the name luminol. Luminol has excellent chemiluminescent properties. It is widely applied in diagnostic assays as a detection means and in forensic medicine, for example for tracing blood spots. In medicine, 5-amino-2,3-dihydro-1,4-phthalazinedione has been developed in the form of a sodium salt. In some countries it is approved for a broad range of acute and chronic inflammatory disorders, including a.o. acute infections of bacterial and viral origin, particularly of the intestinal tract, hepatitis B and C, gastroenteritis, inflammations such as prostatitis, endometriosis, throat inflammation, bronchial asthma, pneumonia, periodontitis, pyelonephritis and autoimmune diseases such as Crohn's disease, ulcerative colitis, lupus erythematosus and scleroderma. Further, there is still a long list of indications in scientific and patent literature in the treatment of which 5-amino-2,3-dihydro-1,4-phthalazinedione sodium salt was allegedly tested or a beneficial use was suggested (cf. WO 2004/041169; WO 2007/018546; WO 2012/127441; WO 2017/202496; WO 2018/082814: a.o.).

For example, the treatment of chronic obstructive pulmonary disease by systemic administration of 5-amino-2,3-dihydro-1,4-phthalazinedione sodium salt has been suggested. However, evidence of therapeutic efficacy by systemic administration is still lacking.

In EP 3416951 A1 a crystalline polymorph of 5-amino-2,3-dihydro-1,4-phthalazinedione and its inhalatory administration with a nebulizer for the treatment of respiratory diseases is disclosed. This document, however, remains silent about mesh nebulizers, the composition of the aerosol and an optimal particle size distribution therein.

In RU 2266119 C2 the use of 5-amino-2,3-dihydro-1,4-phthalazinedione sodium salt or potassium salt or a mixture thereof in the inhalatory treatment of tuberculosis is described. This document, however, remains silent by which inhalation method and device the substance was administered nor about the composition and parameters of the aerosol used for this purpose.

5-amino-2,3-dihydro-1,4-phthalazinedione sodium salt for use in the prophylaxis or treatment of inflammatory pulmonary diseases by inhalatory administration of a nebulized aerosol, however, has not been described yet.

While most conventional immunomodulatory drugs show serious adverse reactions, or are at least problematic in long-term treatment, 5-amino-2,3-dihydro-1,4-phthalazinedione and its pharmaceutically acceptable salts are well tolerated and have a high safety margin in respect to administered dosages.

To ensure a better solubility and bioavailability pharmaceutically acceptable salts of 5-amino-2,3-dihydro-1,4-phthalazinedione are used. Sodium, potassium and lithium salts have been described for therapeutic applications (cf. WO 2010/082858). Crystal structures for lithium, sodium, potassium, rubidium and cesium salts were described in Guzei et al. (2013) Journal of Coordination Chemistry 66, 3722-3739. Thus, the present patent application refers also to the use of all pharmaceutically acceptable salts of 5-amino-2,3-dihydro-1,4-phthalazinedione.

5-amino-2,3-dihydro-1,4-phthalazinedione is often used as a hydrate, for example as sodium salt dihydrate. Thus, the present patent application refers also to the use of all hydrates and other solvates of 5-amino-2,3-dihydro-1,4-phthalazinedione and its pharmaceutically acceptable salts. 5-amino-2,3-dihydro-1,4-phthalazinedione and its pharmaceutically acceptable salts may build complexes with suitable ligands. Thus, the present patent application refers also to such complexes.

In order to ensure a reproducible and standardized API production and to provide improved stability features of an active agent anhydrous formulations are often preferred. Anhydrate forms of 5-amino-2,3-dihydro-1,4-phthalazinedione sodium salt have been described as crystalline polymorphs in WO 2011/107295 (Form I, Form II) and WO 2016/096143 (Form III). These crystalline polymorphs are virtually free from phase impurities and were characterized by means of X-ray powder diffraction. This method yields a set of characteristic d-values indicating interplanar spacings [Å] and of the corresponding 2-theta (2θ) angles [°] under which Bragg reflections occur. This yields a unique and unambiguous fingerprint of the respective polymorphs.

For Form I the following values were determined:
d values: 13.5; 6.9; 5.2; 4.6; 3.9; 3.5; 3.4; 3.3; 3.1; 3.0 and/or
2-theta values: 6.5; 12.7; 16.9; 19.3; 22.8; 25.8; 26.6; 27.2; 28.7; 30.3.

Form II is characterized by the following values:
d values: 12.9; 7.9; 7.1; 6.5; 5.3; 4.0; 3.7; 3.6; 3.3; 3.2 and/or
2-theta values: 6.8; 11.2; 12.5; 13.7; 16.7; 22.4; 24.3; 24.9; 27.2; 27.8.

Form III yielded the following values:
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93.

5-amino-2,3-dihydro-1,4-phthalazinedione itself shows also polymorphism. A Form I (Paradies (1992) Ber. Bunsen-Ges. Phys. Chem 96: 1027-1031) and a Form II (WO 2017/140430) have been disclosed.

Thus, the present patent application refers also to the use according to the invention of all crystalline forms and polymorphs thereof of 5-amino-2,3-dihydro-1,4-phthalazinedione and its pharmaceutically acceptable salts.

Similar therapeutic effects are known for a variety of phthalazinediones, respectively of derivatives of 5-amino-2,3-dihydro-1,4-phthalazinedione and its pharmaceutically acceptable salts. An example is 6-amino-2,3-dihydrophthalazine-1,4-dione (isoluminol). An overview of suitable phthalazinediones is given in WO 2007/018546. It is reasonable to assume that these compounds show comparable effects when being used for the therapeutic applications according to the invention.

Tautomerism relates to a rapid intraconversion of organic compounds in which a hydrogen atom or proton formally migrates inside the compound. This is accompanied by a switch of a single bond and adjacent double bond. The single forms are called tautomers. For example, keto-enol tautomerism occurs in 5-amino-2,3-dihydro-1,4-phthalazinedione (Proescher and Moody (1939) J Lab Clin Med, 1183-1189). Thus, the present patent application refers also to the use of all tautomers of 5-amino-2,3-dihydro-1,4-phthalazinedione and its pharmaceutically acceptable salts.

Isomer is a generic term for molecules with the same chemical formula but a different chemical structure. They can be differentiated into constitutional (structural) isomers (wherein an exchange of atoms or of a functional group occurs) and stereoisomers. Stereoisomers can be subdivided into enantiomers (non-superimposable mirror images of the same molecule) and diastereomers (the same molecule with a different configuration at one or more stereocenters). Diastereomers can be subdivided into cis/trans isomers (referring to the relative orientation of functional groups within a molecule) and on the other hand conformers (rotation about formally single bonds) and rotamers (different rotational positioning about a single bond). An example for a constitutional isomer of 5-amino-2,3-dihydro-1,4-phthalazinedione is 6-amino-2,3-dihydrophthalazine-1,4-dione (isoluminol). Stereoisomers may occur in phthalazinedione derivatives. Thus, the present patent application refers also to the use of all isomers of 5-amino-2,3-dihydro-1,4-phthalazinedione, its derivatives and pharmaceutically acceptable salts.

For some applications it may be desirable that isotopically enriched forms of the compounds of the invention are used, e.g. for diagnostic purposes. Thus, the present patent application refers also to such isotopically enriched forms of the compounds of the invention.

From a pharmacokinetic point of view or for a production rationale it may be preferable to use a prodrug as a dosage form. A prodrug is administered in a pharmacologically inactive form and is metabolically converted into the active form inside the body. This conversion may occur systemically or locally. Thus, the present patent application refers also to prodrugs of the compounds of the invention.

As used throughout the present application the term "5-amino-2,3-dihydro-1,4-phthalazinedione or its pharmaceutically acceptable salts" shall encompass all the aforementioned molecular variants of 5-amino-2,3-dihydro-1,4-phthalazinedione, i.e. 5-amino-2,3-dihydro-1,4-phthalazinedione or one of its pharmaceutically acceptable salts or solvates, hydrates, crystalline polymorphs, tautomers or isotopically enriched forms thereof.

In the scope of this application an aerosol is a mixture of air and solid or liquid particles. In particular, the term "aerosol containing 5-amino-2,3-dihydro-1,4-phthalazinedione or one of its pharmaceutically acceptable salts" refers to an aerosol that has been generated by nebulization of an aqueous solution containing 5-amino-2,3-dihydro-1,4-phthalazinedione or one of its pharmaceutically acceptable salts.

Unless otherwise stated, any technical or scientific term used in the present invention has the meaning that a man skilled in the relevant technical art will attribute to them.

According to the application the terms "drug substance", "active substance", "active agent", "pharmaceutically active agent", "active ingredient" or "active pharmaceutical ingredient" (API) refer to 5-amino-2,3-dihydro-1,4-phthalazinedione or its pharmaceutically acceptable salts, if not stated otherwise or used in a general sense.

The terms "composition" or "pharmaceutical composition" comprise at least one active ingredient in any pharmacologically acceptable defined dosage and dosage form together with at least one pharmaceutically acceptable excipient, as well as all agents that are generated from the ingredients as outlined below directly or indirectly as a combination, accumulation, complex or crystal, or as a consequence of other reactions or interactions, as well as optionally at least one further pharmaceutical drug, as listed below.

The term "excipient" is used in this application to describe any component of a pharmaceutical composition apart of the pharmaceutically active principle. The selection of suitable excipients depends on a variety of factors, such as the dosage form, the dosage, the desired solubility and the stability of the composition.

The terms "effect", "therapeutic effect", "action", "therapeutic action", "efficacy" and "effectiveness" in regard to the substance of the invention or any other active substance mentioned in the description refers to causally occurring beneficial consequences in the organism to which said substance has been administered before.

According to the invention the terms "effective amount" and "therapeutically effective amount" refer to an amount of the substance of the invention that is sufficiently large to cause a desired beneficial effect in a subject in need of such a treatment.

The terms "treatment" and "therapy" comprise the administration of at least the substance of the invention, alone or in combination with at least one further pharmaceutical drug, independently of the chronological order of the administration. Such an administration is intended to substantially improve the disease course of an inflammatory pulmonary disease by either completely curing the disease or by stopping or decelerating the increase of disabilities during the course of the disease.

The terms "prophylaxis" or "prophylactic treatment" comprise the administration of at least the substance of the invention, alone or in combination with at least one further pharmaceutical drug, independently of the chronological order of the administration, in order to prevent or suppress the manifestation of symptoms attributed to an inflammatory pulmonary disease. It refers in particular to medical conditions of a patient in which the manifestation of such symptoms is expected to occur in the far or near future with a reasonable probability.

The terms "subject" and "patient" comprise individuals suffering from disease symptoms or disabilities related to an inflammatory pulmonary disease wherein said diagnosis is either approved or suspected. Individuals are mammals, in particular humans.

In the scope of the present application the term "medicine" shall comprise human and veterinary medicine.

In the sense of the present patent application the terms "inflammatory diseases" or "inflammatory pulmonary diseases" refer to diseases, disorders or other body conditions in which an inflammation, in particular of the lungs, becomes manifest as a major symptom. An inflammation is the response of body tissues to irritation (exogenous or endogenous noxae) or injury. It can be provoked amongst others by physical, chemical and biologic stimuli, comprising mechanical trauma, radiation damage, corrosive chemicals, extremes of heat or cold, infectious agents such as bacteria, viruses, fungi and other pathogenic microorganisms or parts of them. An inflammation can have beneficial (e.g. within the scope of wound healing) and/or detrimental effects in the affected tissue(s). At a first stage an inflammation is regarded as acute. When it isn't terminated after some time the inflammation may become chronic. Typical signs of an inflammation are redness, swelling, heat development, pain and reduced functionality. This may even lead to a loss of function of the affected tissue.

An inflammation is one of the first responses of the immune system that has become activated e.g. by an infection or degenerated endogenous cells. The system of innate immunity mediates an unspecific response, amongst others a general inflammatory response, while the adaptive immune system provides reactions specific to the respective pathogen, which will then be remembered by the immune system. An organism can be in an immunodeficient state, i.e. the immune response is not able to cope with the aforementioned irritations or injuries in a satisfactory manner. On the other hand, the immune system might become hyperactive and turn its defense against endogenous tissues, as in the case of autoimmune diseases.

In the sense of the present patent application the terms "degenerative diseases" or "degenerative pulmonary diseases" refer to diseases, disorders or other body conditions in which a continuous process leads to degenerative cell changes. The affected tissues or organs deteriorate continuously over time. Such a degeneration may be due to physical or physiological over-exercise of specific vulnerable body structures, lifestyle, eating habits, age, congenital diseases or other endogenous causes. The degeneration can be caused or accompanied by an atrophy or dystrophy of the respective tissue or organ, especially the lungs. Often a loss of function and/or an irreversible damage of the affected tissue or organ occurs.

In the sense of the present patent application the terms "lesion", "microlesion" and "trauma" refer to injuries of different size and scope in the affected pulmonary tissue. They can be inflicted by spontaneous physical impact in which the impacting force or torque leads to a tissue damage. But they can also be the final consequence of a previous degenerative disease of the affected pulmonary tissue, or vice versa a microlesion may be the starting point of such a degenerative disease in the wake of the microlesion. Also an inflammation of the affected pulmonary tissue can favor such a microlesion or trauma, or it can be their sequelae. So these terms are interconnected with inflammation and degenerative disease.

In the sense of the present patent application the term "primary" disease, as e.g. a "primary inflammatory or degenerative disease" refers to pulmonary diseases which are not autoimmune-mediated.

If it is known that a healthy person is or will be vulnerable to inflammatory or degenerative diseases, or a tissue damage is to be expected due to a constant overstrain of the respective tissue or organ it can be indicated to give a prophylactic medication in order to prevent or at least to mitigate the expected impairment or damage. Thus, the present patent application refers also to a prophylactic use according to the invention.

There are also cases in which an inflammatory pulmonary disease results in a degenerative disease. Examples therefore will be given further below. Thus, the present patent application refers to the use according to the invention in the prophylaxis and/or treatment of inflammatory and/or degenerative pulmonary diseases, particularly in the treatment of primary inflammatory and/or degenerative pulmonary diseases.

In the scope of the present application the term "pulmonary" refers to organs and tissues of the lower respiratory tract. Examples of organs and tissues of the lower respiratory tract are, without being limiting, the lungs including their lobes, apices, lingulae and alveoli; the bronchi including respiratory bronchioles; tracheal and bronchi rings including the carina; pulmonary vessels including lung vessels and bronchial vessels and bronchial vessels; bronchopulmonary lymph nodes; autonomous nervous system of the lung;
In the scope of the present application the "pulmonary" further refers to adjacent organs and tissues that functionally or structurally are closely linked to the lower respiratory tract and/or the thorax and therefore can be pharmaceutically accessed excellently by inhalation. Examples are, without being limiting, pleura and diaphragm.

In the scope of the present application the terms "alveoli" and "alveolar" refer to the tissue structures at the bottom of the lung airways. Alveoli are hollow cup-shaped cavities found in the lung parenchyma where gas exchange takes place. Further, they are located sparsely on the respiratory bronchioles, line the walls of the alveolar ducts, and are more numerous in the blind-ended alveolar sacs. The alveolar membrane is the gas exchange surface, surrounded by a network of capillaries. Across the membrane oxygen is diffused into the capillaries and carbon dioxide released from the capillaries into the alveoli to be breathed out. Alveoli consist of an epithelial layer of simple squamous epithelium and an extracellular matrix surrounded by capillaries. The epithelial lining is part of the alveolar membrane, also known as the respiratory membrane.

Type I and type II pneumocytes are found in the alveolar wall. Alveolar macrophages are immune cells that move about in the alveolar lumen and in the connective tissue between them. Type I cells are squamous epithelial cells, thin and flat and form the structure of the alveoli. Type II cells (goblet cells) release pulmonary surfactant to lower surface tension.

A typical pair of human lungs contain about 300 million alveoli, producing 70 m² of surface area. Each alveolus is wrapped in a fine mesh of capillaries covering about 70% of its area. The diameter of a typical healthy alveolus is between 200 and 500 µm.

In Example 1 an *ex vivo* mouse lung was exposed over 5 minutes to cigarette smoke. Cigarette smoke is known to contain a plethora of cytotoxic agents that a.o. lead to a drastic intracellular increase of reactive oxygen species (ROS) and reactive nitrogen species (RNS) in the affected lung tissue. ROS/RNS are known to cause multiple cellular damages such as radical formation of further cellular molecules, peroxide formation, lipid peroxidation, damage of the cell membrane and intracellular membranes, DNA damages, unwanted protein modifications and induction of apoptosis. On the other hand, they are key mediators for initiating an immune response that helps the cell, respectively the tissue or the whole organism to cope with xenobiotics (e.g. toxins from cigarette smoke) and infections. The problem is an overshooting immune reaction that causes the aforementioned symptoms of an acute or chronic inflammation. Therefore, it is generally acknowledged that the reduction of excessive intracellular ROS/RNS levels is a promising approach for an anti-inflammatory therapy in general. Specifically, this also holds true for inflammatory pulmonary diseases. Hence the *ex vivo* lung model used in Example 1 is not only indicative for therapeutic efficacy in pulmonary diseases caused by cigarette smoke such as COPD but for all inflammatory pulmonary diseases.

The administration of 5-amino-2,3-dihydro-1,4-phthalazinedione sodium salt to this cigarette smoke-stimulated mouse lung showed a dose-dependent reduction of ROS/RNS levels down to control levels before cigarette smoke exposition. This proves that 5-amino-2,3-dihydro-1,4-phthalazinedione and in particular 5-amino-2,3-dihydro-1,4-phthalazinedione sodium salt has a huge potential for prophylactic and therapeutic efficacy in inflammatory pulmonary diseases.

Inflammatory pulmonary diseases can be classified as follows (according to ICD-10 Chapter X: Diseases of the respiratory system (J00-J99), Version 2016, as of January 10^{th}, 2020):

### a) Inflammation of the lower airways due to a bacterial, viral, fungal or parasitic infection

These diseases comprise, without being limiting, influenza due to identified avian influenza virus; influenza with pneumonia, influenza virus identified; influenza with other respiratory manifestations, influenza virus identified; influenza with other manifestations, influenza virus identified; influenza with pneumonia, virus not identified; influenza with other respiratory manifestations, virus not identified; influenza with other manifestations, virus not identified; adenoviral pneumonia; pneumonia due to *Streptococcus* pneumoniae; pneumonia due to *Haemophilus* influenzae; pneumonia due to *Klebsiella* pneumoniae; pneumonia due to *Pseudomonas;* pneumonia due to *Staphylococcus;* pneumonia due to *Streptococcus,* group B; pneumonia due to other streptococci; pneumonia due to *Escherichia coli;* pneumonia due to other aerobic Gram-negative bacteria; pneumonia due to *Mycoplasma pneumoniae;* other bacterial pneumonia; bacterial pneumonia, unspecified; chlamydial pneumonia; pneumonia due to other specified infectious organisms; pneumonia in bacterial diseases classified elsewhere; pneumonia in viral diseases classified elsewhere; pneumonia in mycoses; pneumonia in parasitic diseases; pneumonia in other diseases classified elsewhere; bronchopneumonia, unspecified; lobar pneumonia, unspecified; hypostatic pneumonia, unspecified; other pneumonia, organism unspecified; pneumonia, unspecified; acute bronchitis; acute bronchiolitis; unspecified acute lower respiratory infection.

### b) Chronic lower respiratory diseases

These diseases comprise, without being limiting, bronchitis, not specified as acute or chronic; simple and mucopurulent chronic bronchitis; chronic bronchitis; chronic tracheitis; chronic tracheobronchitis; emphysema; chronic obstructive pulmonary disease (COPD); asthma; status asthmaticus; bronchiectasis; sarcoidosis of the lung; pulmonary alveolar microlithiasis.

### c) Lung diseases due to an external agent

These diseases comprise, without being limiting, coalworker's pneumoconiosis; asbestosis; pneumoconiosis due to talc dust; silicosis; aluminosis of lung; bauxite fibrosis of lung; berylliosis; graphite fibrosis of lung; siderosis; stannosis; pneumoconiosis due to other specified inorganic dusts; unspecified pneumoconiosis; pneumoconiosis associated with tuberculosis; byssinosis; flax-dresser's disease; cannabinosis; airway disease due to other specific organic dusts; farmer's lung; bagassosis; bird fancier's lung; suberosis; maltworker's lung; mushroom-worker's lung; maple-bark-stripper's lung; air-conditioner and humidifier lung; hypersensitivity pneumonitis due to other organic dusts such as cheese-washer lung, coffee-worker lung, fishmeal-worker lung, furrier lung and sequiosis; hypersensitivity pneumonitis due to unspecified organic dust such as allergic alveolitis and hypersensitivity pneumonitis; respiratory conditions due to inhalation of chemicals, gases, fumes and vapors; pneumonitis due to solids and liquids; radiation pneumonitis; fibrosis of lung following radiation; acute drug-induced interstitial lung disorders; chronic drug-induced interstitial lung disorders; drug-induced interstitial lung disorders, unspecified; respiratory conditions due to other specified external agents; respiratory conditions due to unspecified external agent.

### d) Respiratory diseases principally affecting the interstitium

These diseases comprise, without being limiting, adult respiratory distress syndrome; pulmonary edema such as cardiogenic pulmonary edema, pulmonary permeability edema and high-altitude pulmonary edema; eosinophilic asthma; Löffler's pneumonia; tropical pulmonary eosinophilia; alveolar and parietoalveolar conditions; Hamman-Rich syndrome; pulmonary fibrosis; idiopathic pulmonary fibrosis; other specified interstitial pulmonary diseases; interstitial pulmonary disease, unspecified.

### e) Suppurative and/or necrotic conditions of lower respiratory tract

These diseases comprise, without being limiting, abscess of lung with pneumonia, pyothorax and empyema.

### f) Pleura diseases

These diseases comprise, without being limiting, pleurisy with effusion; pleural effusion in conditions classified elsewhere; pleural plaque; pneumothorax; chylothorax; fibrothorax; hemothorax; hemopneumothorax; hydrothorax; pleural condition, unspecified.

### g) Postprocedural or related lower respiratory diseases

These diseases comprise, without being limiting, acute pulmonary insufficiency following thoracic surgery; acute pulmonary insufficiency following nonthoracic surgery; chronic pulmonary insufficiency following surgery; host-versus-graft disease after lung transplantation; graft-versus-host disease after lung transplantation; chronic lung allograft dysfunction (CLAD), chronic lung allograft dysfunction - bronchiolitis obliterans syndrome (CLAD-BOS); lung ischemia reperfusion injury; primary graft dysfunction after lung transplantation; Mendelson's syndrome; other postprocedural respiratory disorders; postprocedural respiratory disorder, unspecified; respiratory failure, not elsewhere classified; diseases of bronchus, not elsewhere classified; pulmonary collapse; atelectasis; interstitial emphysema; mediastinal emphysema; compensatory emphysema; mediastinitis; disorders of diaphragm.

### h) Pulmonary diseases specific to the perinatal period

These diseases comprise, without being limiting, respiratory distress syndrome of newborn; transient tachypnoea of newborn; congenital pneumonia due to viral agent; congenital pneumonia due to *Chlamydia;* congenital pneumonia due to *Staphylococcus;* congenital pneumonia due to *Streptococcus,* group B; congenital pneumonia due to *Escherichia coli;* congenital pneumonia due to *Pseudomonas;* congenital pneumonia due to bacterial agents such as *Haemophilus influenzae, Klebsiella pneumoniae, Mycoplasma, Streptococcus,* except group B; congenital pneumonia due to other organisms; congenital pneumonia, unspecified; neonatal aspiration of meconium; interstitial emphysema originating in the perinatal period; pneumothorax originating in the perinatal period; pneumomediastinum originating in the perinatal period; other conditions related to interstitial emphysema originating in the perinatal period; pulmonary hemorrhage originating in the perinatal period; Wilson-Mikity syndrome; bronchopulmonary dysplasia originating in the perinatal period; unspecified chronic respiratory disease originating in the perinatal period.

### i) Trauma and injuries of the lower respiratory tract and/or the thorax

These conditions comprise, without being limiting, injury of pulmonary blood vessels; traumatic pneumothorax; traumatic hemothorax; traumatic hemopneumothorax; other injuries of lung; injury of bronchus; injury of pleura; injury of diaphragm; crushing injury of thorax and traumatic amputation of part of thorax.

### j) Malignant neoplasms of the lower respiratory tract

These diseases comprise, without being limiting, malignant neoplasm of bronchus and lung; lung cancer; non-small-cell lung cancer; adenocarcinoma; squamous-cell lung carcinoma; large-cell lung carcinoma; pulmonary enteric adenocarcinoma; bronchioloalveolar carcinoma; ovine pulmonary adenocarcinoma; small-cell lung cancer; bronchial leiomyoma; bronchial carcinoma; Pancoast tumor; pulmonary carcinoid tumor; pleuropulmonary blastoma; neuroendocrine tumors of the lung; lymphomas of the lung; lymphangiomatosis of the lung; sarcomas of the lung; alveolar soft part sarcoma; vascular tumors of the lung; mediastinal tumors; pleural tumors; metastasis to the lung.

In detail, the present application refers to 5-amino-2,3-dihydro-1,4-phthalazinedione or its pharmaceutically acceptable salts for use in the prophylaxis or treatment of inflammatory pulmonary diseases by inhalatory administration, wherein the inflammatory pulmonary disease is selected from the group comprising inflammations of the lower airways due to a bacterial, viral, fungal or parasitic infection, chronic lower respiratory diseases, lung diseases due to an external agent, respiratory diseases principally affecting the interstitium, suppurative and/or necrotic conditions of lower respiratory tract, pleura diseases, postprocedural or related lower respiratory diseases, pulmonary diseases specific to the perinatal period, trauma and injuries of the lower respiratory tract and/or the thorax, and malignant neoplasms of the lower respiratory tract.

In particular, the present application refers to 5-amino-2,3-dihydro-1,4-phthalazinedione or its pharmaceutically acceptable salts for use in the prophylaxis or treatment of inflammatory pulmonary diseases by inhalatory administration, wherein the inflammatory pulmonary disease is an inflammation of the lower airways due to a bacterial, viral, fungal or parasitic infection.

In particular, the present application refers to 5-amino-2,3-dihydro-1,4-phthalazinedione or its pharmaceutically acceptable salts for use in the prophylaxis or treatment of inflammatory pulmonary diseases by inhalatory administration, wherein the inflammatory pulmonary disease is a chronic lower respiratory disease.

In particular, the present application refers to 5-amino-2,3-dihydro-1,4-phthalazinedione or its pharmaceutically acceptable salts for use in the prophylaxis or treatment of inflammatory pulmonary diseases by inhalatory administration, wherein the inflammatory pulmonary disease is a lung disease due to an external agent.

In particular, the present application refers to 5-amino-2,3-dihydro-1,4-phthalazinedione or its pharmaceutically acceptable salts for use in the prophylaxis or treatment of inflammatory pulmonary diseases by inhalatory administration, wherein the inflammatory pulmonary disease is a respiratory diseases principally affecting the interstitium.

In particular, the present application refers to 5-amino-2,3-dihydro-1,4-phthalazinedione or its pharmaceutically acceptable salts for use in the prophylaxis or treatment of inflammatory pulmonary diseases by inhalatory administration, wherein the inflammatory pulmonary disease is a suppurative and/or necrotic condition of the lower respiratory tract.

In particular, the present application refers to 5-amino-2,3-dihydro-1,4-phthalazinedione or its pharmaceutically acceptable salts for use in the prophylaxis or treatment of inflammatory pulmonary diseases by inhalatory administration, wherein the inflammatory pulmonary disease is a pleura disease.

In particular, the present application refers to 5-amino-2,3-dihydro-1,4-phthalazinedione or its pharmaceutically acceptable salts for use in the prophylaxis or treatment of inflammatory pulmonary diseases by inhalatory administration, wherein the inflammatory pulmonary disease is a postprocedural or related lower respiratory disease.

In particular, the present application refers to 5-amino-2,3-dihydro-1,4-phthalazinedione or its pharmaceutically acceptable salts for use in the prophylaxis or treatment of inflammatory pulmonary diseases by inhalatory administration, wherein the inflammatory pulmonary disease is a pulmonary disease specific to the perinatal period.

In particular, the present application refers to 5-amino-2,3-dihydro-1,4-phthalazinedione or its pharmaceutically acceptable salts for use in the prophylaxis or treatment of inflammatory pulmonary diseases by inhalatory administration, wherein the inflammatory pulmonary disease is a condition due to a trauma and/or injury of the lower respiratory tract and/or the thorax.

In particular, the present application refers to 5-amino-2,3-dihydro-1,4-phthalazinedione or its pharmaceutically acceptable salts for use in the prophylaxis or treatment of inflammatory pulmonary diseases by inhalatory administration, wherein the inflammatory pulmonary disease is a malignant neoplasm of the lower respiratory tract.

In the scope of the present application COPD is of particular interest. COPD is a progressive development of airflow limitation that is not fully reversible. Most COPD patients suffer from three pathological conditions: Bronchitis, emphysema and mucus plugging. This disease is characterized by a slowly progressive and irreversible decrease in forced expiratory volume in the first second of expiration (FEV1), with relative preservation of forced vital capacity (FVC). In both asthma and COPD there is a significant but distinct remodeling of the airways. Most of the airflow obstruction is due to two major components, alveolar destruction (emphysema) and small airways obstruction (chronic obstructive bronchitis). COPD is mainly characterized by profound mucus cell hyperplasia. A primary characteristic of COPD is neutrophil infiltration into the patient's lungs. Elevated levels of proinflammatory cytokines, like TNF-alpha, and especially chemokines like interleukin-8 (IL-8) play a prominent role in the pathogenesis of COPD. Platelet thromboxane synthesis was found to be enhanced in COPD patients. Most of the tissue damage is caused by activation of neutrophils followed by their release of matrix metalloproteinases, and increased production of ROS and RNS.

Emphysema describes the destruction of the lung architecture with enlargement of the airspaces and loss of alveolar surface area. Lung damage is caused by weakening and breaking the air sacs within the lungs. Several adjacent alveoli may rupture, forming one large space instead of many small ones. Larger spaces can combine into an even bigger cavity, called a bulla. As a result, natural elasticity of the lung tissue gets lost, leading to overstretching and rupture, thus minimizing lung compliance. There is also less pull on the small bronchial tubes, which can cause their collapse and obstruct the airflow. Air not exhaled before the next breath cycle gets trapped in the lungs, leading to shortage of breath. The sheer effort it takes to force air out of the lungs upon exhaling is exhausting for the patients.

The most common symptoms of COPD include shortness of breath, chronic cough, chest tightness, greater effort to breathe, increased mucus production and frequent clearing of the throat. Patients become unable to perform their usual daily activities.

Long-term smoking is the most common cause of COPD, responsible for 80-90 % of all cases. Other risk factors are heredity, second-hand smoke, air pollution, and a history of frequent childhood respiratory infections. COPD is progressive and sometimes irreversible; there is currently no cure.

The clinical development of COPD is typically described in three stages:
Stage 1: Lung function (as measured by FEV1) is greater than or equal to 50% of predicted normal lung function. There is minimal impact on health-related quality of life. Symptoms may progress during this stage, and patients may begin to experience severe breathlessness, requiring evaluation by a pulmonologist.
Stage 2: FEV1 lung function is 35 to 49% of predicted normal lung function, and there is a significant impact on health-related quality of life.
Stage 3: FEV1 lung function is less than 35% of predicted normal lung function, and there is a profound impact on health-related quality of life.

Symptomatic pharmaceutical therapy includes the administration of bronchodilators, glucocorticoids and PDE4 inhibitors. Suitable bronchodilators are e.g. beta-2 adrenergic agonists such as the short-acting fenoterol and salbutamol as well as the long-acting salmeterol and formoterol, muscarinic anticholinergics such as ipratropium bromide and tiotropium bromide, and methylxanthines such as theophylline.

Suitable glucocorticoids include inhalatory glucocorticoids such as budesonide, beclometasone and fluticasone, orally administered glucocorticoids such as prednisolone and intravenously administered glucocorticoids such as prednisolone.

A suitable PDE (phosphodiesterase) 4 inhibitor is roflumilast.

Thus, the present application refers also to 5-amino-2,3-dihydro-1,4-phthalazinedione or its pharmaceutically acceptable salts for use in the prophylaxis or treatment of COPD by inhalatory administration.

In the scope of the present application also asthma is of particular interest. Asthma is a chronic inflammatory disease of the lower airways. It is mainly characterized by recurring symptoms such as reversible airflow obstruction and easily triggered bronchospasms. Symptoms include episodes of wheezing, coughing, chest tightness and dyspnea.

Asthma is thought to be caused by a combination of genetic and environmental factors. Environmental factors include exposure to air pollution and allergens. Other potential triggers may be iatrogenic.

Asthma is clinically classified into intermittent, mild persistent, moderate persistent and severe persistent, based on the frequency of symptoms. The most important parameters are FEV1 and peak expiratory flow rate.

Until now asthma cannot be cured. For long-term therapy, symptoms such as status asthmaticus can be prevented by avoiding triggers, such as allergens and irritants, and pharmacologically by the use of inhaled corticosteroids. Long-acting beta-2 agonists (LABA) or antileukotriene agents may be used additionally. The most common inhaled corticosteroids include beclomethasone, budesonide, fluticasone, mometasone and ciclesonide. Suitable LABA include salmeterol and formoterol. Leukotriene receptor antagonists such as montelukast, pranlukast and zafirlukast are orally administered. Suitable 5-lipooxygenase (5-LOX) inhibitors include meclofenamate sodium and zileuton. In severe stages of asthma intravenous corticosteroids such as prednisolone are recommended.

Acute asthma seizures (status asthmaticus) are best treated with inhaled short-acting beta-2 agonists such as salbutamol. Ipratropium bromide can be inhaled additionally. Intravenously, corticosteroids can be administered.

Inhalatory administration is commonly effected through metered-dose inhalers, respectively dry-powder inhalers.

Thus, the present application refers also to 5-amino-2,3-dihydro-1,4-phthalazinedione or its pharmaceutically acceptable salts in the prophylaxis or treatment of asthma by inhalatory administration.

In the scope of the present application also sarcoidosis of the lung is of particular interest. Sarcoidosis of the lung (interchangeably used herein: pulmonary sarcoidosis, PS) is characterized by abnormal collections of inflammatory cells that form lumps known as granulomas in the lung. The cause of sarcoidosis is unknown. The disease usually begins in the lungs, skin or lymph nodes, and can become manifest throughout the body. The most common symptom is a long-lasting fatigue, even if the disease activity has ceased. Overall malaise, shortness of breath, joint complaints, temperature increase, weight loss and skin complaints may be present. In general, the prognosis is good. Especially the acute form usually causes few problems, as the complaints will gradually decrease by themselves. If sarcoidosis is present in the heart, kidneys, liver and/or central nervous system, or extensively manifest in the lungs the outcome is less favorable. In general, sarcoidosis is classified in four stages determined by chest radiography. However, these stages do not correlate with the grade of severity. 1. bihilar lymphadenopathy (granulomas in the lymph nodes); 2. bihilar lymphadenopathy and reticulonodular infiltrates (granulomas in the lungs); 3. bilateral pulmonary infiltrates (granulomas in the lungs, but not in the lymph nodes); 4. fibrocystic sarcoidosis typically with upward hilar retraction, cystic and bullous changes (irreversible scarring in the lungs, i.e. pulmonary fibrosis). Stage 2 and 3 patients often display a chronic progressive disease course.

Symptomatic pharmaceutical therapy of sarcoidosis of the lung includes the administration of corticosteroids such as prednisone and prednisolone, immunosuppressive agents such as TNF-alpha inhibitors (etanercept, adalimumab, golimumab, infliximab), cyclophosphamide, cladribine, cyclosporine, chlorambucil and chloroquine, IL-23 inhibitors such as tildrakizumab and guselkumab, antimetabolites such as mycophenolic acid, leflunomide, azathioprine and methotrexate. In subtypes such as Löfgren's syndrome COX inhibitors such as acetylsalicylic acid, diclofenac or ibuprofen are used. All these active agents are administered systemically until now.

Thus, the present application refers also to 5-amino-2,3-dihydro-1,4-phthalazinedione or its pharmaceutically acceptable salts for use in the prophylaxis or treatment of sarcoidosis of the lung by inhalatory administration.

In the scope of the present application also cystic fibrosis is of particular interest. Cystic fibrosis is an inherited form of chronic bronchitis with mucus hypersecretion, generally accompanied by poor clearance of the airway secretions, obstruction of airflow and chronic bacterial infection of the airways, commonly by *Pseudomonas aeruginosa.* It is known that the sputum and bronchoalveolar lavage fluid from cystic fibrosis patients reduce the ability of neutrophils to kill these bacteria. Obstruction of the airways by such secretions can cause respiratory distress, and in some cases, can lead to respiratory failure and death. Further symptoms include sinus infections, poor growth, fatty stool, clubbing of the fingers and toes, and male infertility.

Cystic fibrosis is an autosomal-recessive hereditary disease caused by mutations in the gene for the cystic fibrosis transmembrane conductance regulator (CFTR) protein. CFTR is involved in the production of sweat, digestive fluids, and mucus. CFTR is a channel protein that controls the flow of H₂O and Cl⁻ ions in and out of cells inside the lungs. When the CFTR protein is working correctly, ions freely flow in and out of the cells. However, when the CFTR protein is malfunctioning, these ions cannot flow out of the cell due to a blocked channel. This causes cystic fibrosis, characterized by the buildup of thick mucus in the lungs.

No cure for cystic fibrosis is known. Intravenous, inhaled, and oral antibiotics are used to treat chronic and acute infections. Mechanical devices and inhalation medications are used to alter and clear the thickened mucus. These therapies, while effective, can be extremely time-consuming. Oxygen therapy at home is recommended in those with significant low oxygen levels. Inhaled antibiotics include e.g. levofloxacin, tobramycin, aztreonam and colistin. Orally administered antibiotics include e.g. ciprofloxacin and azithromycin. Mutation-specific CFTR potentiators are ivacaftor and tezacaftor.

Thus, the present application refers also to 5-amino-2,3-dihydro-1,4-phthalazinedione or its pharmaceutically acceptable salts for use in the prophylaxis or treatment of cystic fibrosis by inhalatory administration.

In the scope of the present application also bronchiectasis is of particular interest. Bronchiectasis is believed to be an idiopathic disease. Morphologically, it is characterized by a permanent enlargement of parts of the lower airways. As pathologic conditions, a.o. post-infection, immune deficiency, exaggerated immune response, congenital abnormalities, inflammatory pneumonitis, fibrosis and mechanical obstruction are discussed. Symptoms include chronic cough along with daily production of mucus. Thus, it resembles cystic fibrosis, but without the characteristic gene mutation. Pulmonary function testing results generally show airflow obstruction ranging from moderate to severe. Additional symptoms include dyspnea, coughing up blood, chest pain, hemoptysis, fatigue, and weight loss.

Treatment of bronchiectasis aims at controlling infections and bronchial secretions, relieving airway obstructions, removal of affected portions of lung by surgery or artery embolization. If indicated, antibiotics, in particular macrolide antibiotics are administered. Mucus overproduction can be addressed by mucolytics. Bronchodilators are used for facilitating breathing. Continuous inhaled corticosteroids help to some extent to reduce sputum production, to decrease airway constriction and to prevent disease progression.

Thus, the present application refers also to 5-amino-2,3-dihydro-1,4-phthalazinedione or its pharmaceutically acceptable salts in the prophylaxis or treatment of bronchiectasis by inhalatory administration.

In the scope of the present application also adult respiratory distress syndrome is of particular interest. Adult respiratory distress syndrome (ARDS) is a respiratory failure syndrome. It can have a variety of causes, such as pneumonia, trauma, severe burns, blood transfusion, aspiration, sepsis, pancreatitis or as a reaction on certain drugs. The gas exchange in the alveoli is seriously impaired due to injury of the alveolar endothelial cells, surfactant dysfunction, overshooting reaction of the immune system and coagulation disorders. A rapid migration of neutrophils and T lymphocytes into the affected lung tissue is observed. Acute symptoms include dyspnea, tachypnea and blue coloration of the skin. If the patient survives lung function is often permanently impaired. Acute treatment is mainly based on mechanical ventilation in intensive care units, and if indicated, the administration of antibiotics. Nitric oxide inhalation may help to improve oxygenation of the blood but has other drawbacks. Extracorporeal membrane oxygenation (ECMO) helps to increase the survival rate. However, pharmaceutical treatment, e.g. with corticosteroids, is controversially discussed.

Thus, the present application refers also to 5-amino-2,3-dihydro-1,4-phthalazinedione or its pharmaceutically acceptable salts for use in the prophylaxis or treatment of adult respiratory distress syndrome by inhalatory administration.

In the scope of the present application also pulmonary fibrosis is of particular interest. Herein scars are formed in the pulmonary tissues, leading to serious breathing problems. Scar formation, respectively the accumulation of excess fibrous connective tissue leads to thickening of the walls, thus causing reduced oxygen supply in the blood. The consequence is a chronic progressive dyspnea. Pulmonary fibrosis is often secondary to other pulmonary diseases, e.g. in interstitial lung disorders, autoimmune diseases of the lung, inhalation of environmental and occupational pollutants, or certain infections. Otherwise, it is classified as idiopathic pulmonary fibrosis.

Pulmonary fibrosis involves gradual exchange of lung parenchyma with fibrotic tissue. The scar tissue causes an irreversible decrease in oxygen diffusion capacity, resulting in stiffness or decreased compliance of the lung. Pulmonary fibrosis is perpetuated by aberrant wound healing.

Up to now, there is no general pharmaceutical treatment of lung fibrosis. Some subtypes are responsive to corticosteroids such as prednisone, to anti-fibrotic agents such as pirfenidone and nintedanib, or to immunosuppressive agents, such as cyclophosphamide, azathioprine, methotrexate, penicillamine, and cyclosporine.

Thus, the present application refers also to 5-amino-2,3-dihydro-1,4-phthalazinedione or its pharmaceutically acceptable salts for use in the prophylaxis or treatment of pulmonary fibrosis, respectively idiopathic pulmonary fibrosis by inhalatory administration.

A typical inflammatory disease caused by an external agent is berylliosis (interchangeably herein, chronic beryllium disease, CBD). There is no cure for this occupational disease, only symptomatic treatment.

Prolonged exposure by inhalation may sensitize the lungs to beryllium, leading to the development of small inflammatory nodules, called granulomas. Typically, CBD granulomas are not characterized by necrosis and therefore not exhibiting a caseating appearance. Ultimately, this process leads to a decrease in pulmonary diffusion capacity. The typical symptoms are cough and dyspnea. Other symptoms include chest pain, joint aches, weight loss, and fever. The patient's T-cells become sensitized to beryllium. The pathologic immune response leads to an accumulation of CD4+ helper T-lymphocytes and macrophages in the lungs. There they aggregate together and form granulomas. Eventually, this leads to lung fibrosis. Treatment options include oxygen application and orally administered corticosteroids.

Thus, the present application refers also to 5-amino-2,3-dihydro-1,4-phthalazinedione or its pharmaceutically acceptable salts for use in the prophylaxis or treatment of berylliosis by inhalatory administration.

In the scope of the present application also chronic lung allograft dysfunction is of particular interest. Chronic lung allograft dysfunction (CLAD), respectively chronic lung allograft dysfunction - bronchiolitis obliterans syndrome (CLAD-BOS) is a major problem in the long-term management of lung transplant recipients. Both alloimmune-dependent factors (rejection) and alloimmune-independent factors contribute to the development of CLAD. It encompasses all forms of chronic pulmonary function decline, after eliminating known causes (persistent acute rejection, infection, anastomotic stricture, or disease recurrence, pleural disease, diaphragm dysfunction or native lung hyperinflation). Therefore, it is a heterogeneous entity in which two main phenotypes are currently identified: Bronchiolitis obliterans syndrome (BOS), defined by a persistent decline in FEV1, and an obstructive functional pattern.

No treatment is currently available to reverse CLAD after diagnosis. Pharmaceutical treatment of the symptoms is carried out with azithromycin (first line), alternatively montelukast. In therapy-resistant cases photopheresis is applied.

Thus, the present application refers also to 5-amino-2,3-dihydro-1,4-phthalazinedione or its pharmaceutically acceptable salts for use in the prophylaxis or treatment of CLAD, respectively CLAD-BOS by inhalatory administration.

In the scope of the present application also pulmonary edema is of particular interest. Pulmonary edema may have different causes. Fluid accumulation occurs in the tissue and air spaces of the lungs, leading to impaired gas exchange and in the worst case to respiratory failure. Therapy for pulmonary edema focusses mainly on maintaining vital functions, e.g. by tracheal intubation and mechanical ventilation. Hypoxia symptoms can be addressed by oxygen supplementation

Cardiogenic pulmonary edema can be a result of congestive heart failure which is due to the heart's inability to pump the blood out of the pulmonary circulation at a sufficient rate resulting in elevation in wedge pressure and pulmonary edema. The underlying causes may be left ventricular failure, arrhythmias, or fluid overload, e.g., from kidney failure or intravenous therapy. It can be also caused by a hypertensive crisis, as the elevation in blood pressure and increased afterload on the left ventricle hinders forward flow and causes the elevation in wedge pressure and subsequent pulmonary edema. In acute cases, a loop diuretic such as furosemide is administered, often together with morphine to reduce respiratory distress. Both diuretic and morphine may have vasodilator effects, but also specific nitric oxide vasodilators such as intravenous glyceryl trinitrate or isosorbide dinitrate may be used.

Pulmonary permeability edema is characterized by reduced alveolar Na⁺ uptake capacity and capillary barrier dysfunction and is a potentially lethal complication, e.g. in listeriosis induced by listeriolysin. Apical Na⁺ uptake is mainly mediated by the epithelial sodium channel (ENaC) and initiates alveolar liquid clearance.

High-altitude pulmonary edema (HAPE) occurs in otherwise healthy people at altitudes typically above 2,500 meters and can be life-threatening. After a rapid gain in altitude, symptoms may include shortness of breath at rest, cough, weakness or decreased exercise performance, chest tightness or congestion, crackles or wheezing, central blue skin color, tachypnea and tachycardia. The lower air pressure at high altitudes leads to a decrease in partial pressure of arterial oxygen. Due to hypoxemia pulmonary hypertension secondary to hypoxic pulmonary vasoconstriction and increased capillary pressure develop. This leads to subsequent leakage of cells and proteins into the alveoli. Hypoxic pulmonary vasoconstriction occurs diffusely, leading to arterial vasoconstriction in all areas of the lung.

The first medical measure is a descent to a lower altitude as quickly as possible. Alternatively, oxygen supplementation for maintaining an S_{pO2} above 90% is possible.

Pharmaceutical prophylaxis of HAPE includes calcium channel blockers such as nifedipine, PDE5 inhibitors such as sildenafil and tadalafil and inhaled beta 2-agonists such as salmeterol.

A new pharmaceutical approach to enhance ENaC function is e.g. the peptide drug solnatide.

Thus, the present application refers also to 5-amino-2,3-dihydro-1,4-phthalazinedione or its pharmaceutically acceptable salts for use in the prophylaxis or treatment of pulmonary edema by inhalatory administration, in particular in the prophylaxis or treatment of cardiogenic pulmonary edema, pulmonary permeability edema and high-altitude pulmonary edema.

In the scope of the present application also lung ischemia reperfusion injury is of particular interest. In lung transplantation, organ ischemia and subsequent reperfusion is unavoidable and commonly leads to acute, sterile inflammation after transplant called ischemia-reperfusion (IR) injury. Severe IR injury leads to primary graft dysfunction (PGD), which is the major source of both short- and long-term morbidity and mortality after lung transplantation. Currently, there are no therapeutic agents clinically utilized to specifically prevent IR injury, and treatment strategies are limited to supportive care. If feasible, the donor lung, respectively the donor can be prophylactically treated with 5-amino-2,3-dihydro-1,4-phthalazinedione or one of its pharmaceutically acceptable salts.

Endothelial cell dysfunction and disruption of the endothelial barrier are hallmarks of lung IR injury. Depolarization of endothelial cell membranes induces ROS production and subsequent inflammation and leukocyte extravasation. Activation of NADPH oxidase (NOX2), induction of nitric oxide (NO) production, and activation of integrin αvβ5, promote vascular permeability via ROS/RNS production. Alveolar macrophages are activated. Elevated chemokine levels and adhesion molecule expression on endothelial cells and neutrophils lead to binding and infiltration of neutrophils, which can release cytokines, ROS and form neutrophil extracellular traps (NETs).

Recent prophylactic strategies before lung transplantation include administration to the organ recipient of anti-oxidants (free radical scavengers) or inhibitors of oxidant-producing enzymes (e.g. methylene blue or N-acetylcysteine), anti-inflammatory strategies using inhibitors of pro-inflammatory transcription factors or inflammatory mediators, ventilation with gaseous molecules such as carbon monoxide or inhaled anesthetic sevoflurane, growth factors or dietary supplements such as creatine, as well as cell-based therapies such as application of mesenchymal stem cells.

Thus, the present application refers also to 5-amino-2,3-dihydro-1,4-phthalazinedione or its pharmaceutically acceptable salts for use in the prophylaxis or treatment of lung ischemia reperfusion injury by inhalatory administration.

In the scope of the present application also primary graft dysfunction after lung transplantation is of particular interest. Primary graft dysfunction (PGD) is a devastating form of acute lung injury that afflicts about 10% to 25% of patients in the first hours to days after lung transplantation. Clinically and pathologically it is a syndrome that mimics adult respiratory distress syndrome (ARDS) and carries a mortality of up to 50%. PGD can have different causes such as ischemia reperfusion injury described before, epithelial cell death, endothelial cell dysfunction, innate immune activation, oxidative stress, release of inflammatory cytokines and chemokines as well as iatrogenic factors such as mechanical ventilation and transfusion of blood components. Activation of the innate immune system activation has been demonstrated during the onset and spread of ischemia reperfusion injury. Herein PGD is associated with the innate immunity pathways of a Toll-like receptor-mediated injury.

Molecular markers of PGD include intracellular adhesion molecule-1, surfactant protein-1, plasminogen activator inhibitor, soluble receptor for advance glycation end products and protein C.

Approaches for avoiding PGD development include reperfusion optimization, regulation of prostaglandin levels, hemodynamic control, hormone replacement, ventilator management and donor lung preparation strategies. To decrease PGD incidence, strategies, such as using prostaglandins, nitric oxide, surfactant, adenosine or inhibition of pro-inflammatory mediators and/or elimination of free oxygen radicals, have been used. Furthermore, for the inhibition of neutrophils and neutrophil-borne mediators, free oxygen radicals, cytokines, proteases, lipid mediators, adhesion molecules and complement cascade inhibitors have been investigated. Inhaled nitric oxide may lower the pulmonary arterial pressure, without affecting the systemic blood pressure. As a last life-saving resort option, extracorporeal membrane oxygenation (ECMO) is used for correcting PGD-induced hypoxemia and by providing necessary gas exchange.

Thus, the present application refers also to 5-amino-2,3-dihydro-1,4-phthalazinedione or its pharmaceutically acceptable salts for use in the prophylaxis or treatment of primary graft dysfunction after lung transplantation by inhalatory administration.

For an effective prophylactic or therapeutic treatment of the aforementioned inflammatory pulmonary diseases 5-amino-2,3-dihydro-1,4-phthalazinedione or its pharmaceutically acceptable salts have to reach the patient's alveoli. Therefore, the particle size must be sufficiently small to reach the lowest parts of the airways of the pulmonary tissue. The best inhalatory device class for inhalatory application of a pharmaceutically active agent are the so-called mesh nebulizers described before. In the scope of the present application practically all mesh nebulizers known in the art can be used, from rather simple single-use mesh nebulizers for cough and cold or for fancy purposes to sophisticated high-end mesh nebulizers for clinical or domestic treatment of serious diseases or conditions of the lower airways.

Suitable commercially available mesh nebulizers, jet nebulizers, ultrasonic nebulizers, dry powder inhalers and (pressurized) metered-dose inhalers comprise, without being limiting, eFlow^{®}rapid, PARI LC STAR^{®}, PARI Velox and PARI Velox Junior (PARI GmbH, Starnberg, Germany), Philips Respironics I-neb and Philips InnoSpire Go (Koninklijke Philips N.V., Eindhoven, Netherlands), VENTA-NEB^{®}-ir, OPTI-NEB^{®}, M-neb^{®} dose⁺ mesh nebulizer inhalation MN-300/8, M-Neb Flow+ and M-neb^{®} mesh nebulizer MN-300/X (NEBU-TEC, Eisenfeld, Germany), Homed Deepro HCM-86C and HCM860 (HCmed Innovations Co., Ltd, Taipei, Taiwan), OMRON MicroAir U22 and U100 (OMRON, Kyoto, Japan), Aerogen^{®} Solo, Aerogen^{®} Ultra and Aerogen^{®} PRO (Aerogen, Galway, Ireland), KTMED NePlus NE-SM1 (KTMED Inc., Seoul, South Korea), Vectura Bayer Breelib^{™} (Bayer AG, Leverkusen, Germany), Vectura Fox, MPV Truma and MicroDrop^{®} Smarty (MPV MEDICAL GmbH, Kirchheim, Germany), MOBI MESH (APEX Medical, New Taipei City, Taiwan), B.Well WN-114, TH-134 and TH-135 (B.Well Swiss AG, Widnau, Switzerland), Babybelle Asia BBU01 (Babybelle Asia Ltd., Hongkong), CA-MI Kiwi and others (CA-MI sri, Langhirano, Italy), Diagnosis PRO MESH (Diagnosis S.A., Bia 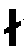 ystok, Poland), DIGI O₂ (DigiO₂ International Co., Ltd., New Taipei City, Taiwan), feellife AIR PLUS, AEROCENTRE+, AIR 360+, AIR GARDEN, AIRICU, AIR MASK, AIRGEL BOY, AIR ANGEL, AIRGEL GIRL and AIR PRO 4 (Feellife Health Inc., Shenzhen, China), Hannox MA-02 (Hannox International Corp., Taipei, Taiwan), Health and Life HL100 and HL100A (HEALTH & LIFE Co., Ltd., New Taipei City, Taiwan), Honsun NB-810B (Honsun Co., Ltd., Nantong, China), K-jump^{®} KN-9100 (K-jump Health Co., Ltd., New Taipei City, Taiwan), microlife NEB-800 (Microlife AG, Widnau, Switzerland), OK Biotech Docspray (OK Biotech Co., Ltd., Hsinchu City, Taiwan), Prodigy Mini-Mist^{®} (Prodigy Diabetes Care, LLC, Charlotte, USA), Quatek NM211, NE203, NE320 and NE403 (Big Eagle Holding Ltd., Taipei, Taiwan), Simzo NBM-1 and NBM-2 (Simzo Electronic Technology Ltd., Dongguan, China), Mexus^{®} BBU01 and BBU02 (Tai Yu International Manufactory Ltd., Dongguan, China), TaiDoc TD-7001 (TaiDoc Technology Co., New Taipei City, Taiwan), Vibralung^{®} and HIFLO Miniheart Circulaire II (Westmed Medical Group, Purchase, USA), KEJIAN (Xuzhou Kejian Hi-Tech Co., Ltd., Xuzhou, China), YM-252, P&S-T45 and P&S-360 (TEKCELEO, Valbonne, France), Maxwell YS-31 (Maxwell India, Jaipur, India), Kernmed^{®} JLN-MB001 (Kernmed, Durmersheim, Germany).

Preferred are mesh nebulizers with a piezoelectric activation of the nebulization process, respectively vibrating mesh nebulizers.

Mesh nebulizers can be classified into two groups according to patient interaction: Continuous mode devices and trigger-activated devices. In continuous mode mesh nebulizers the nebulized aerosol is continuously released into the mouth piece and the patient has to inhale the provided aerosol. In trigger-activated devices a defined amount of aerosol is released only upon an active and deep inspiratory breath. This way a far larger amount of active agent-containing aerosol is inhaled and reaches the lowest airways than with continuous mode devices. The latter lose a large amount of active agent-containing aerosol either to the surrounding or on the passage of the upper airways, as the aerosol release is not coupled to the respiratory cycle.

Therefore, trigger-activated mesh nebulizers are preferred, in particular vibrating mesh nebulizers.

Particularly preferred are trigger-activated mesh nebulizers with a piezoelectric activation of the nebulization process.

Preferred are the mesh nebulizer models PARI eFlow^{®}rapid, Philips Respironics I-neb, Philips InnoSpire Go, M-neb^{®} dose⁺ mesh nebulizer inhalation MN-300/8, Homed Deepro HCM-86C and HCM860, OMRON MicroAir U100, Aerogen^{®} Solo, KTMED NePlus NE-SM1, Vectura Fox, Vectura Bayer Breelib^{™}.

The most preferred vibrating mesh nebulizer models are high-end models such as PARI eFlow^{®}rapid, PARI Velox, Philips Respironics I-neb, M-neb^{®} dose⁺ mesh nebulizer inhalation MN-300/8, Aerogen^{®} Solo, Vectura Fox, Vectura Bayer Breelib^{™}.

Thus, the present application refers also to 5-amino-2,3-dihydro-1,4-phthalazinedione or one of its pharmaceutically acceptable salts for use in the prophylaxis or treatment of inflammatory pulmonary diseases by inhalatory administration, wherein a mesh nebulizer releases for the inhalatory administration an aerosol containing liquid droplets of 5-amino-2,3-dihydro-1,4-phthalazinedione or one of its pharmaceutically acceptable salts. This also holds true for the aforementioned subtypes of inflammatory pulmonary diseases as well as for the aforementioned single inflammatory pulmonary diseases, respectively.

The mean droplet size is usually characterized as MMAD (median mass aerodynamic diameter). The individual droplet size is referred to as MAD (mass aerodynamic diameter). This value indicates the diameter of the nebulized particles (droplets) at which 50% are smaller or larger, respectively. Particles with a MMAD > 10 µm normally don't reach the lower airways, they often get stuck in the throat. Particles with a MMAD > 5 µm and < 10 µm usually reach the bronchi but not the alveoli. Particles between 100 nm and 1 µm MMAD don't deposit in the alveoli and are exhaled immediately. Therefore, the optimal range is between 1 µm and 5 µm MMAD. Recent publications even favor a narrower range between 3.0 µm and 4.0 µm (cf. Amirav et al. (2010) J Allergy Clin Immunol 25: 1206-1211; Haidl et al. (2012) Pneumologie 66: 356-360).

Therefore, the MMAD of nebulized 5-amino-2,3-dihydro-1,4-phthalazinedione or one of its pharmaceutically acceptable salts should be between 3.0 µm and 4.0 µm, preferably between 3.0 µm and 3.5 µm, more preferred between 3.0 and 3.4 µm, even more preferred between 3.0 µm and 3.3 µm, and most preferred between 3.0 µm and 3.2 µm.

Likewise, at least 50% of the liquid droplets of the aerosol according to the invention should have a MAD between 1 µm and 5 µm. Preferentially, at least 50% of the liquid droplets of the aerosol according to the invention should have a MAD between 1 µm and 4 µm, further preferred at least 50% of the liquid droplets of the aerosol according to the invention should have a MAD between 1 µm and 3.5 µm, most preferred at least 50% of the liquid droplets of the aerosol according to the invention should have a MAD between 1 µm and 3.3 µm.

A further commonly accepted quality parameter is the percentage of the particles in the generated aerosol with a diameter in the range of 1 µm to 5 µm (FPM; fine particle mass). FPM is a measure for the particle distribution. It is calculated by subtracting the percentage of the particles in the generated aerosol with a diameter in the range < 1 µm from the overall percentage of the particles in the generated aerosol with a diameter in the range < 5 µm (FPF; fine particle fraction).

Therefore, the FPM of nebulized 5-amino-2,3-dihydro-1,4-phthalazinedione or one of its pharmaceutically acceptable salts should be at least 50%, preferably at least 55 %, more preferred at least 60%, and most preferred at least 65%.

It is expressly stated that the values and ranges disclosed herein refer to measurements of the MMAD, FPF and FPM by means of a cascade impactor (Next Generation Impactor, NGI) cooled down to 4°C.

In order to test whether the combination of a mesh nebulizer and 5-amino-2,3-dihydro-1,4-phthalazinedione sodium salt is able to meet this objective the FPM in a thus generated aerosol was determined. As laid out in Example 2, 5 ml of a 20 mg/ml physiological saline solution of 5-amino-2,3-dihydro-1,4-phthalazinedione sodium salt, originally prepared from Form I (see above) were nebulized. It was surprisingly found that under these conditions 69.0 % (FPM) of the particles were in the targeted range. This is a much more favorable particle size distribution percentage as found for many other pharmaceutically active agents. This percentage may of course slightly vary according to the selected aqueous solution, temperature, mesh nebulizer model, selected piezoelectric excitation frequency, outlet geometry and dosage per application. Further, it was surprisingly found in the same experiment that the MMAD for this nebulization is 3.1 µm. Thus, this MMAD meets perfectly the desired range.

It is understood that the MMAD for this nebulization may slightly vary according to conditions such as ambient temperature, temperature of the pharmaceutical formulation to be nebulized, concentrations of the pharmaceutically active agent, optional choice of excipients etc.

The present application refers also to 5-amino-2,3-dihydro-1,4-phthalazinedione or one of its pharmaceutically acceptable salts for use in the prophylaxis or treatment of inflammatory pulmonary diseases by inhalatory administration, wherein a mesh nebulizer releases for the inhalatory administration an aerosol containing liquid droplets of 5-amino-2,3-dihydro-1,4-phthalazinedione or one of its pharmaceutically acceptable salts, and at least 50% of the liquid droplets of the aerosol are in the size range of 1 µm to 5 µm in diameter.

In a more preferred embodiments at least 55% of the liquid droplets of the aerosol are in the size range of 1 µm to 5 µm in diameter.

In a more preferred embodiments at least 60% of the liquid droplets of the aerosol are in the size range of 1 µm to 5 µm in diameter.

In particularly preferred embodiments at least 65% of the liquid droplets of the aerosol are in the size range of 1 µm to 5 µm in diameter.

The present application refers also to 5-amino-2,3-dihydro-1,4-phthalazinedione or one of its pharmaceutically acceptable salts for use in the prophylaxis or treatment of inflammatory pulmonary diseases by inhalatory administration, wherein a mesh nebulizer releases for the inhalatory administration an aerosol containing liquid droplets of 5-amino-2,3-dihydro-1,4-phthalazinedione or one of its pharmaceutically acceptable salts, and the mass median aerodynamic diameter of these droplets is in the range of 3 µm to 4 µm.

In preferred embodiments the mass median aerodynamic diameter of these droplets is in the range of 3 µm to 3.5 µm.

In more preferred embodiments the mass median aerodynamic diameter of these droplets is in the range of 3 µm to 3.4 µm.

In even more preferred embodiments the mass median aerodynamic diameter of these droplets is in the range of 3 µm to 3.3 µm.

In particularly preferred embodiments the mass median aerodynamic diameter of these droplets is in the range of 3 µm to 3.2 µm.

Thus the present application refers to 5-amino-2,3-dihydro-1,4-phthalazinedione or one of its pharmaceutically acceptable salts for use in the prophylaxis or treatment of inflammatory pulmonary diseases by inhalatory administration, wherein a mesh nebulizer releases for the inhalatory administration an aerosol containing liquid droplets of 5-amino-2,3-dihydro-1,4-phthalazinedione or one of its pharmaceutically acceptable salts, and said aerosol is characterized in that the fine particle mass is at least 50% of the liquid droplets of the aerosol.

Thus the present application refers to 5-amino-2,3-dihydro-1,4-phthalazinedione or one of its pharmaceutically acceptable salts for use in the prophylaxis or treatment of inflammatory pulmonary diseases by inhalatory administration, wherein a mesh nebulizer releases for the inhalatory administration an aerosol containing liquid droplets of 5-amino-2,3-dihydro-1,4-phthalazinedione or one of its pharmaceutically acceptable salts, and said aerosol is characterized in that the median mass aerodynamic diameter of the liquid droplets of the aerosol is between 3.0 µm and 3.3 µm.

Thus the present application refers to 5-amino-2,3-dihydro-1,4-phthalazinedione or one of its pharmaceutically acceptable salts for use in the prophylaxis or treatment of inflammatory pulmonary diseases by inhalatory administration, wherein a vibrating mesh nebulizer releases for the inhalatory administration an aerosol containing liquid droplets of 5-amino-2,3-dihydro-1,4-phthalazinedione or one of its pharmaceutically acceptable salts, and the median mass aerodynamic diameter of the liquid droplets of the aerosol is between 3.0 µm and 3.3 µm, and said aerosol is characterized in that the fine particle mass is at least 50% of the liquid droplets of the aerosol, wherein the fine particle mass indicates the percentage of the liquid droplets with a diameter in the range of 1.0 to 5.0 µm.

In preferred embodiments said aerosol is characterized in that the median mass aerodynamic diameter of the liquid droplets of the aerosol is between 3.0 µm and 3.2 µm.

In another aspect, the present application refers to 5-amino-2,3-dihydro-1,4-phthalazinedione or one of its pharmaceutically acceptable salts for use in an aerosol for inhalatory administration for the prophylaxis or treatment of inflammatory pulmonary diseases, wherein the median mass aerodynamic diameter of the liquid droplets of the aerosol is between 3.0 µm and 3.3 µm, and said aerosol is characterized in that the fine particle mass is at least 50% of the liquid droplets of the aerosol, wherein the fine particle mass indicates the percentage of the liquid droplets with a diameter in the range of 1.0 to 5.0 µm.

In preferred embodiments the aforementioned aerosol is characterized in that the median mass aerodynamic diameter of the liquid droplets of the aerosol is between 3.0 µm and 3.2 µm.

This also holds true for the aforementioned subtypes of inflammatory pulmonary diseases as well as for the aforementioned single inflammatory pulmonary diseases, respectively.

Preferentially, the present application refers to the aforementioned embodiments, wherein 5-amino-2,3-dihydro-1,4-phthalazinedione sodium salt is used.

More preferred, the present application refers to the aforementioned embodiments, wherein crystalline polymorphic Forms I, II or III of 5-amino-2,3-dihydro-1,4-phthalazinedione sodium salt as defined above by their respective d values and/or 2-theta values determined by means of x-ray powder diagrams are used.

Most preferred, the present application refers to the aforementioned embodiments, wherein crystalline polymorphic Form I of 5-amino-2,3-dihydro-1,4-phthalazinedione sodium salt as defined above by their respective d values and/or 2-theta values determined by means of x-ray powder diagrams is used.

In another aspect of the invention the present application refers to an aerosol produced by a mesh nebulizer, containing 5-amino-2,3-dihydro-1,4-phthalazinedione or one of its pharmaceutically acceptable salts in the range of 0.01 % per weight to 10 % per weight,

an aqueous solution in the range of 70 % per weight to 99.99 % per weight, and optionally at least one pharmaceutically acceptable excipient in the range of 0 % per weight to 20 % per weight, wherein said percentages add up to 100 %.

In particular, the present application refers to an aerosol produced by a vibrating mesh nebulizer, containing 5-amino-2,3-dihydro-1,4-phthalazinedione or one of its pharmaceutically acceptable salts in the range of 0.01% per weight to 10 % per weight, an aqueous solution in the range of 70 % per weight to 99.99 % per weight, and optionally at least one pharmaceutically acceptable excipient in the range of 0 % per weight to 20 % per weight, wherein said percentages add up to 100 %, the median mass aerodynamic diameter of the liquid droplets of the aerosol is between 3.0 µm and 3.3 µm and said aerosol is characterized in that the fine particle mass is at least 50% of the liquid droplets of the aerosol, wherein the fine particle mass indicates the percentage of the liquid droplets with a diameter in the range of 1.0 to 5.0 µm.

In another aspect of the invention the present application refers also to a pharmaceutical composition for use in the prophylaxis or treatment of inflammatory pulmonary diseases, comprising an aerosol produced by a nebulizer from an aqueous solution,
containing 5-amino-2,3-dihydro-1,4-phthalazinedione or one of its pharmaceutically acceptable salts in the range of 0.01 % per weight to 10 % per weight, an aqueous solution in the range of 70 % per weight to 99.99 % per weight, and
optionally at least one pharmaceutically acceptable excipient in the range of 0 % per weight to 20 % per weight, wherein said percentages add up to 100 %.

In another aspect of the invention the present application refers also to a method of treatment of inflammatory pulmonary diseases, comprising the steps of
a) providing an aerosol according to the invention by nebulization with a mesh nebulizer, and
b) administering a therapeutically effective amount of said aerosol to a patient in need thereof via a mouthpiece for inhalation fitting to said mesh nebulizer via self-inhalation by the patient.

The formulations of 5-amino-2,3-dihydro-1,4-phthalazinedione or its pharmaceutically acceptable salts may contain at least one pharmaceutically acceptable excipient.

The term "pharmaceutical excipients" refers to natural or synthetic compounds that are added to a pharmaceutical formulation alongside the pharmaceutical active agent. They may help to bulk up the formulation, to enhance the desired pharmacokinetic properties or the stability of the formulation, as well as being beneficial in the manufacturing process. Advantageous classes of excipients according to the invention include colorants, buffers, preservatives, antioxidants, pH regulators, solvents, isotonizing agents, opacifiers, aromatic and flavoring substances.

Colorants are excipients that bestow a colorization to the pharmaceutical formulation. These excipients can be food colorants. They can be adsorbed on a suitable adsorption means such as clay or aluminum oxide. A further advantage of a colorant is that it may visualize spilled aqueous solution on the nebulizer and/or the mouthpiece to facilitate cleaning. The amount of the colorant may vary between 0.01 and 10 % per weight of the pharmaceutical composition, preferred between 0.05 and 6 % per weight, more preferred between 0.1 and 4 % per weight, most preferred between 0.1 and 1 % per weight.

Suitable pharmaceutical colorants are for example curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulphite caramel, ammonia caramel, sulphite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Moreover, buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid, maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)methylaminol] propane), ethylene diamine, ACES (2-[(amino-2-oxoethyl)amino]ethanesulfonic acid), imidazole, MOPS (3-(N-morphino)-propanesulfonic acid, diethyl malonic acid, TES (2-[tris(hydroxymethyl)methyl]aminoethanesulfonic acid, HEPES (N-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazole, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), 4-morpholino-propanesulfonic acid (MOPS) and N,N-bis-(2-hydroxyethyl)-2-aminoethanesulfonic acid (BES). Another group of preferred buffers are glycine, glycyl-glycine, glycyl-glycyl-glycine, N,N-bis-(2-hydroxyethyl)glycine and N-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, N,N,N-trimethyllysine, 3-methyl histidine, 5-hydroxy-lysine, o-phosphoserine, gamma-carboxyglutamate, [epsilon]-N-acetyl lysine, [omega]-N-methyl arginine, citrulline, ornithine and their derivatives.

Preservatives for liquid and/or solid dosage forms can be used on demand. They may be selected from the group comprising, but not limited to, sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulfite, sodium bisulfite, sodium metabisulfite, potassium metabisulfite, potassium sulfite, calcium sulfite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

Suitable solvents may be selected from the group comprising, but not limited to, water, carbonated water, water for injection, water with isotonizing agents, saline, isotonic saline, alcohols, particularly ethyl and n-butyl alcohol, and mixtures thereof.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

The addition of a sufficient amount of antioxidants is particularly preferable for liquid dosage forms. Suitable examples for antioxidants include sodium metabisulfite, alpha-tocopherol, ascorbic acid, maleic acid, sodium ascorbate, ascorbyl palmitate, butylated hydroxyanisol, butylated hydroxytoluene, fumaric acid or propyl gallate. Preferred is the use of alpha-tocopherol and ascorbyl palmitate.

Suitable pH-regulators for liquid dosage forms are e.g. sodium hydroxide, hydrochloric acid, buffer substances such as sodium dihydrogen phosphate or disodium hydrogenphosphate.

Suitable aromatic and flavoring substances comprise above all essential oils that can be used for this purpose. In general, this term refers to volatile extracts from plants or parts of plants with the respective characteristic smell. They can be extracted from plants or parts of plants by steam distillation.

Suitable examples are: Essential oils, respectively aromatic substances from sage, cloves, chamomile, anise, star anise, thyme, tea tree, peppermint, mint oil, menthol, cineol, borneol, zingerol, eucalyptus oil, mango, figs, lavender oil, chamomile blossoms, pine needles, cypress, oranges, rosewood, plum, currant, cherry, birch leaves, cinnamon, limes, grapefruit, tangerine, juniper, valerian, lemon balm, lemon grass, palmarosa, cranberry, pomegranate, rosemary, ginger, pineapple, guava, echinacea, ivy leave extract, blueberry, kaki, melons etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

Opacifiers are substances that render the liquid dosage for, opaque, if desired. They must have a refractive index substantially different from the solvent, in most cases here water. At the same time they should be inert to the other components of the composition. Suitable examples include titanium dioxide, talc, calcium carbonate, behenic acid, cetyl alcohol, or mixtures thereof.

According to the invention all of the aforementioned excipients and classes of excipients can be used without limitation alone or in any conceivable combination thereof, as long as the inventive use is not thwarted, toxic actions may occur or the respective national legislations are infracted.

In another aspect of the invention the present application refers also to a method for producing an aerosol according to the invention, comprising the following steps:
a) filling 0.1 ml to 5 ml of an aqueous solution containing 5-amino-2,3-dihydro-1,4-phthalazinedione or one of its pharmaceutically acceptable salts and optionally at least one pharmaceutically acceptable excipient into the nebulization chamber of a mesh nebulizer,
b) starting vibration of the mesh of the mesh nebulizer at a frequency of 80 kHz to 200 kHz, and
c) discharging the generated aerosol at the side of the mesh of the mesh nebulizer opposite to the nebulization chamber.

The vibration frequency of vibrating mesh nebulizers is normally in the range of 80 kHz to 200 kHz, preferred 90 kHz to 180 kHz, more preferred 100 kHz to 160 kHz, most preferred 105 kHz to 130 kHz (cf. Chen, *The Aerosol Society:* **DDL2019;** Gardenshire et al. (2017) A Guide to Aerosol Delivery Devices for Respiratory Therapists, 4th ed.).

Thus, the aforementioned method is also disclosed with said vibration frequency ranges.

As can be seen in the aerosol analysis shown in Example 2, the method of the invention proved to be particularly effective in nebulizing a high percentage of the pharmaceutically active agent from the provided aqueous solution. Therefore, there is a relatively small loss of the pharmaceutically active agent during the nebulization step.

The method according to the invention is thus characterized in that at least 80 % in weight, preferred at least 85 % in weight, most preferred at least 90 % in weight of 5-amino-2,3-dihydro-1,4-phthalazinedione or one of its pharmaceutically acceptable salts contained in said aqueous solution are nebulized in the generated aerosol.

As can be seen in the aerosol analysis and the respective experimental settings of Example 2, the method of the invention proved to be particularly effective in nebulizing a high percentage of the pharmaceutically active agent from the provided aqueous solution during a short time. This is an important feature for patient compliance. A considerable percentage of the patient population finds the inhalatory process to be uncomfortable, weary and physically demanding. On the other hand, the patient's active cooperation is essential for an effective and targeted inhalatory application. Therefore, it is desirable that a therapeutically sufficient amount is applied during a period of time as short as possible. Surprisingly, it showed that during a three minutes time span 95 % of the substance provided in the aqueous solution could be nebulized. This is an ideal time span for a high patient compliance.

Therefore, the method according to the invention is thus characterized in that at least 80 % of the generated aerosol are produced during three minutes after starting nebulization in the mesh nebulizer, preferred at least 85 % and most preferred at least 90 %.

While the pharmaceutically active agent is usually provided in a single dosage container for every nebulization procedure the nebulizer and/or the mouthpiece can be used over a certain period of time and have to be replaced at certain intervals. A cleaning of the nebulizer and the mouthpiece is recommended by default after each nebulization. But herein patient compliance cannot be reasonably taken for granted. But even after a meticulous cleaning there are always some deposits of the aerosol in the nebulization chamber, the outlet and/or the mouthpiece. As the aerosol is produced from an aqueous solution these depositions bear the risk of producing a bioburden of bacteria that might contaminate the inhaled aerosol. Deposits may also plug holes in the mesh membrane of the mesh nebulizer. In general, the nebulizer and/or the mouthpiece should be exchanged every one or two weeks. Therefore, it is convenient to offer the medication and the nebulizer as a combined product.

Thus, in another aspect of the invention the present application refers also to a kit as defined by the claims, comprising a vibrating mesh nebulizer and a pharmaceutically acceptable container with an aqueous solution containing 5-amino-2,3-dihydro-1,4-phthalazinedione or one of its pharmaceutically acceptable salts and optionally at least one pharmaceutically acceptable excipient.

In particular, the present application refers to a kit, comprising a vibrating mesh nebulizer and a pharmaceutically acceptable container with an aqueous solution containing 5-amino-2,3-dihydro-1,4-phthalazinedione or one of its pharmaceutically acceptable salts and optionally at least one pharmaceutically acceptable excipient, wherein said vibrating mesh nebulizer is able to produce an aerosol from said aqueous solution that is characterized in that the median mass aerodynamic diameter of the liquid droplets of the aerosol is between 3.0 µm and 3.3 µm, and in that the fine particle mass is at least 50% of the liquid droplets of the aerosol, wherein the fine particle mass indicates the percentage of the liquid droplets with a diameter in the range of 1.0 to 5.0 µm.

In an alternative kit 5-amino-2,3-dihydro-1,4-phthalazinedione or one of its pharmaceutically acceptable salts is not provided in form of an aqueous solution but in two separated containers, one for a solid form for the active agent and the other for an aqueous solution. The final aqueous solution is freshly prepared by solving the active agent in the final solution. Thereupon the final aqueous solution is filled into the nebulization chamber of the mesh nebulizer. These two containers can be completely separated containers, e.g. two vials, or e.g. a dual-chamber vial. For solving the active agent e.g. a membrane between the two chambers is perforated to allow for mixing of the content of both chambers.

Thus, the present application discloses also a kit, comprising a vibrating mesh nebulizer, a first pharmaceutically acceptable container with water for injection or physiological saline solution and a second pharmaceutically acceptable container with a solid form of 5-amino-2,3-dihydro-1,4-phthalazinedione or one of its pharmaceutically acceptable salts, wherein optionally at least one pharmaceutically acceptable excipient is contained in the first pharmaceutically acceptable container and/or the second pharmaceutically acceptable container.

In particular, the present disclosure refers to a kit, comprising a vibrating mesh nebulizer, a first pharmaceutically acceptable container with water for injection or physiological saline solution and a second pharmaceutically acceptable container with a solid form of 5-amino-2,3-dihydro-1,4-phthalazinedione or one of its pharmaceutically acceptable salts, wherein optionally at least one pharmaceutically acceptable excipient is contained in the first pharmaceutically acceptable container and/or the second pharmaceutically acceptable container, and said vibrating mesh nebulizer is able to produce an aerosol from a solution resulting from the mixing of the content of the first container and the second container, and said aerosol is characterized in that the median mass aerodynamic diameter of the liquid droplets of the aerosol is between 3.0 µm and 3.3 µm, and in that the fine particle mass is at least 50% of the liquid droplets of the aerosol, wherein the fine particle mass indicates the percentage of the liquid droplets with a diameter in the range of 1.0 to 5.0 µm.

The aerosol generated by the method according to the invention is administered, respectively self-administered by means of a mouthpiece. Optionally, such a mouthpiece can be additionally included in the beforementioned kits.

A common way to transfer the provided aqueous solution or final aqueous solution into the nebulization chamber of the mesh nebulizer by means of a syringe equipped with an injection needle. First, the aqueous solution is drawn up into the syringe and then injected into the nebulization chamber. Optionally, such a syringe and/or injection needle can be additionally included in the beforementioned kits. Without being limiting, typical syringes made of polyethylene, polypropylene or cyclic olefin co-polymers can be used, and a typical gauge for a stainless steel injection needle would be in the range of 14 to 27.

### EXAMPLES

5-amino-2,3-dihydro-1,4-phthalazinedione sodium salt Form I was provided by MetrioPharm Deutschland GmbH, Berlin, Germany. Salbutamol and ipratropium bromide were purchased from Merck KGaA, Darmstadt, Germany.

### Example 1:

5-amino-2,3-dihydro-1,4-phthalazinedione sodium salt Form I was tested in an isolated, ventilated and perfused mouse lung system that was stimulated with cigarette smoke.

The isolated, ventilated and perfused mouse lung system (ILU) is an established model for studying acute effects of various conditions and drugs on lung parenchyma and vasculature. It is mainly used for examining effects of hypoxia and for evaluating the efficacy of potential drugs on the hypoxic pulmonary vasoreaction (cf. Weissmann et al. (2006) Proc Natl Acad Sci USA 103: 19093-19098). Results from this experimental set-up are regarded as not only indicative for the treatment of COPD but to all inflammatory disorders of the lower airways.

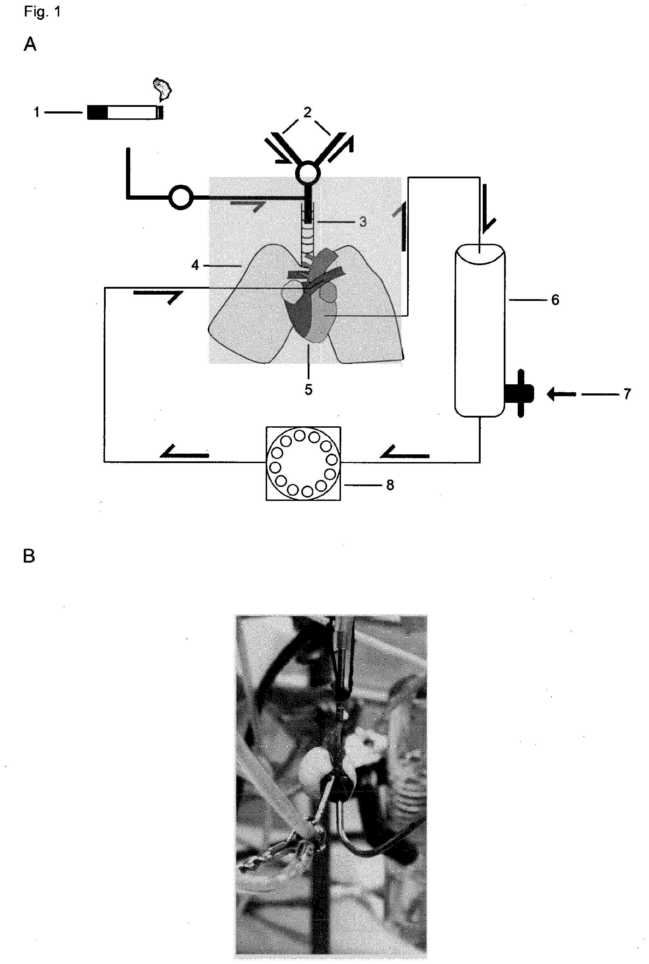

C57BU6J mice (n = 25, 5 per group; male/female, 3-6 months, 20 - 30 g; Charles River GmbH, Sulzfeld, Germany) were anesthetized with a ketamine (100 mg/kg body weight) and xylazine (20 mg/kg body weight) intraperitoneal injection (Ceva Tiergesundheit GmbH, Düsseldorf, Germany) containing heparin (50 I.E. heparin/g body weight; Ratiopharm GmbH, Ulm, Germany). The lungs and the heart were removed from the chest cavity and placed on the ILU system (see Fig. 1A and 1B). Lungs were ventilated in an isolated chamber using normoxic gas (21% O₂, 5% CO₂, 74% N₂; 150 breaths per minute at the PEEP (positive end-expiratory pressure) of 3 cm H₂O) and perfused with a modified Krebs-Henseleit buffer (120.0 mM NaCl, 4.3 mM KCI, 1.1 mM KH₂PO₄, 2.4 mM CaCl₂, 1.3 mM MgCl₂, 13.14 mM glucose, 0.25 mM hydroxyethyl starch 200000/0.5, 25.0 mM NaHCOs adjusted to a constant pH range of 7.37-7.40, 800 mM L-arginine; Serag-Wissner GmbH & Co. KG, Naila, Germany) at a temperature of 37°C. Lung weight, right and left ventricle pressure, as well as ventilatory pressure were monitored and recorded during the whole experimental procedure. After 5-10 minutes, when the lung was properly flushed and all the parameters were stable, 5-amino-2,3-dihydro-1,4-phthalazinedione sodium salt was applied by adding 150 µl of a stock solution to 15 ml of the circulating perfusion buffer. The substance was applied 10 minutes prior to the first cigarette smoke application. Cigarette smoke was applied via trachea while lung is perfused with the buffer containing 5-amino-2,3-dihydro-1,4-phthalazinedione sodium salt. Cigarette smoke was prepared freshly before each application, by burning one cigarette (research cigarettes 3R4F, University of Kentucky, USA) in one minute using normoxic gas at the flow of 1l/min and collected in a 1 I glass bottle containing 5 g silica gel for removing the moist from the cigarette smoke. 50 ml of the cigarette smoke were taken via a syringe and applied to the lung via trachea (Figure 1A) in deep breaths (periodic inflation for 3-4 s) over a period of 5 min. The application was done manually while carefully monitoring the inspiratory pressure to avoid damage of the lung. The cigarette smoke application was repeated three times with a 1 hour break in-between.

Five treatment groups (n = 5, respectively) were investigated:
A: room air exposure
B: cigarette smoke + diluent (buffer solution)
C: cigarette smoke + 0.5 mM 5-amino-2,3-dihydro-1,4-phthalazinedione sodium salt
D: cigarette smoke + 1 mM 5-amino-2,3-dihydro-1,4-phthalazinedione sodium salt
E: cigarette smoke + 2 mM 5-amino-2,3-dihydro-1,4-phthalazinedione sodium salt

5-amino-2,3-dihydro-1,4-phthalazinedione sodium salt Form I was dissolved in water for injection (vehicle) at the required concentrations described above. Stock solutions were prepared in water for injection. Further 1:100 dilutions were made with modified Krebs-Henseleit buffer (see above). Stock solutions were stored at -70°C in appropriate aliquots. The required amount of stock solution was thawed, and the corresponding working solution was prepared for immediate use.

One hour after the third cigarette smoke application lungs were removed from the system and fixed by inflating with formalin solution (via trachea) at the pressure of 12-15 cm H₂O for two hours at room temperature. Afterwards the fixed lungs were kept in PBS (phosphate buffer saline, see below) at +4°C until further dehydration and paraffin embedding. Paraffin blocks were cut 3 µm thick, dried overnight at 37°C and stained for 3-nitrotyrosine (3-NT).

The toxins and xenobiotics in the cigarette smoke lead to a dramatic increase in reactive oxygen species (ROS) and reactive nitrogen species (RNS). Oxidative and nitrosative stress correlate with the severity of inflammatory pulmonary diseases. They elevate the inflammatory response, cause a disbalance of proteolytic and anti-proteolytic activities, augment the number of apoptotic cells and decrease proliferation. These oxidants are able to overwhelm the antioxidant defenses and initiate inflammation by various mechanisms (Foronjy and D'Armiento (2006) Clinical and Applied Immunology Reviews 6: 53-72). The most potent RNS peroxynitrite (ONOO⁻) is formed by reaction between nitric oxide (NO) and superoxide anion radical (O₂⁻) (Szabo et al. (2007) Nat Rev Drug Discov 6: 662-680). ONOO⁻preferably attacks tyrosine residues in proteins to form the stable adduct 3-nitrotyrosine (Ricciardolo et al. (2004) Physiol Rev 84: 731-765; Seimetz et al. (2011) Cell 147: 293-305; Tsoumakidou et al. (2005) Chest 127: 1911-1918). Levels of 3-NT in sputum proteins have been found to negatively correlate with FEV1 in COPD patients (Ricciardolo et al. (2004) Physiol Rev 84: 731-765; Tsoumakidou et al. (2005) Chest 127: 1911-1918). Nitrated tyrosine residues alter cellular signalling, suggesting that 3-NT is not only a marker of nitrosative stress but may also have a functional relationship with the pathophysiology of inflammatory airway diseases (Davis et al. (2002); J Virol 76: 8347-8359; Murata and Kawanishi (2004) Biochem Biophys Res Comm 316: 123-128; Sugiura et al. (2004) Free Radic Res 38: 49-57;). It has been proposed that 3-NT contributes to airway hyper-responsiveness and epithelial damage (Tsoumakidou et al. (2005) Chest 127: 1911-1918) and plays a major role in the development of airway remodeling (Ichinose et al. (2000) Am J Respir Crit Care Med 162: 701-706).

The immunohistochemical staining of 3-nitrotyrosine was carried out according to the following protocol:

| **incubation time** | **reagent/condition** | **process** |
|---|---|---|
| 60 min | 59°C | deparaffinisation |
| 3x 10 min | xylol | |
| 2x 5 min | ethanol 99.6% | |
| 5 min | ethanol 96% | rehydratation |
| 5 min | ethanol 70% | |
| 20 min | 6% hydrogen peroxide in methanol | |
| 4 x 3 min | aqua dest | |
| 45 min | cooking in Rodent decloaker buffer (10x) | antigen retrieval |
| 30 min | cooling down | |
| wash | aqua dest | |
| 2x 5 min | PBS pH 7.4 | |
| 60 min | 10% BSA + 1:1000 DR Fc block | |
| 4x 5 min | PBS pH 7.4 | blocking |
| 30 min | rodent M | |
| 2x 5 min | PBS pH 7.4 | |
| overnight +4°C | primary antibody (nTyr Sigma) 1:200 DR | |
| 4x 5 min | TBS pH 7.2 | |
| 20 min | post block AP | staining |
| 2x 5 min | TBS pH 7.2 | |
| 30 min | polymer AP kit (rabbit/mouse) | |

| | | |
|---|---|---|
| 3× 5 min | TBS pH 7.2 | |
| 1 min | aqua dest | |
| 5-10 min | Warp Red | |
| wash | TBS pH 7.2 | |
| 2 min | hematoxylin 1:10 DR | |
| wash | aqua dest | |
| 1 min | PBS pH 7.4 | |
| 5 min | DAPI in PBS 1:1000 | |
| 2x 2min | PBS pH 7.4 | |
| - | Dako Fluorescent Mounting Medium | covering |

Xylol was purchased from Carl Roth GmbH + Co.KG, Karlsruhe, Germany. Ethanol (96 % and 99.6 %) was purchased from Otto Fischar GmbH % Co.KG, Saarbrücken, Germany. Ethanol (70 %) was purchased from SAV Liquid Production GmbH, Flintsbach am Inn, Germany. Hydrogen peroxide was purchased from Merck KGaA, Darmstadt, Germany. Methanol, Bovine Serum Albumin (BSA), DAPI (4',6-diamidino-2-phenylidone) and Anti-Nitrotyrosine Antibody (N0409; batch: 120M4825) were purchased from Sigma-Aldrich Co., Darmstadt, Germany. Rodent decloaker buffer (10x) and Warp Red Chromogen Kit were purchased from Biocare Medical, Pacheco, Ca., USA. Tris wash buffer (TBS), CAT hematoxylin staining solution and AP Polymer System (mouse/rabbit) were purchased from Zytomed Systems GmbH, Berlin, Germany. Dako Fluorescent Mounting Medium was purchased from Dako North America Inc., Via Real Carpinteria, Ca., USA. TruStain fcX (antimouse CD16/32; DR Fc block) was purchased from BioLegend Inc., San Diego, Ca., USA. PBS (phosphate-buffered saline) was prepared with 8 g/l sodium chloride (Carl Roth GmbH + Co.KG, Karlsruhe, Germany), 0.2 g/l potassium chloride (Carl Roth GmbH + Co.KG, Karlsruhe, Germany), 1.42 g/l disodium hydrogenphosphate (Merck KGaA, Darmstadt, Germany) and 0.27 g/l potassium dihydrogenphosphate (Merck KGaA, Darmstadt, Germany).

Stained histological samples were blindly analysed by light microscope. 3-nitrotyrosine levels in the lung parenchyma were quantified as a percentage of stained surface area. Quantification was performed under 200x magnification in 5-10 randomly selected fields with exclusion of large bronchi and vessels. One-Way ANOVA statistical test with Bonferroni correction was performed for comparison between groups. Differences with p < 0.05 were considered statistically significant.

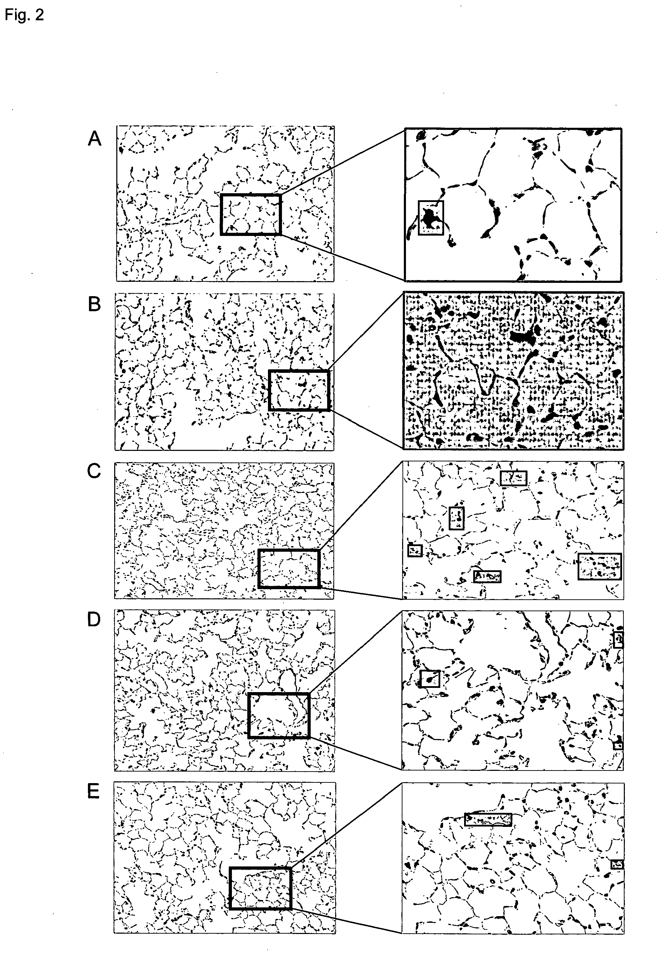

Cigarette smoke applied via trachea lead to a significant increase in 3-nitrotyrosine in septa of the exposed lungs (Fig. 2B), in comparison to room air as control (Fig. 2A). 5-amino-2,3-dihydro-1,4-phthalazinedione sodium salt was added to the perfusing buffer prior to the cigarette smoke application and was kept during the whole experiment. Cigarette smoke induced 3-nitrotyrosine formation could be almost completely abolished in the lungs perfused with buffer containing 1 mM 5-amino-2,3-dihydro-1,4-phthalazinedione sodium salt (Fig. 2D) or 2 mM 5-amino-2,3-dihydro-1,4-phthalazinedione sodium salt (Fig. 2E), whereas the lowest 5-amino-2,3-dihydro-1,4-phthalazinedione sodium salt concentration (0.5mM; Fig. 2C) yielded a moderate effect.

### Quantification of the staining:

| **group** | **mean ± SEM [% area]** |
|---|---|
| A: room air | 0.24 ± 0.09 |
| B: cigarette smoke | 3.07 ± 0.39 |
| C: cigarette smoke + 0.5 mM 5-amino-2,3-dihydro-1,4-phthalazinedione sodium salt | 2.24 ± 0.28 |
| D: cigarette smoke + 1 mM 5-amino-2,3-dihydro-1,4-phthalazinedione sodium salt | 0.72 ± 0.26 |
| E: cigarette smoke + 2 mM 5-amino-2,3-dihydro-1,4-phthalazinedione sodium salt | 0.37 ± 0.13 |

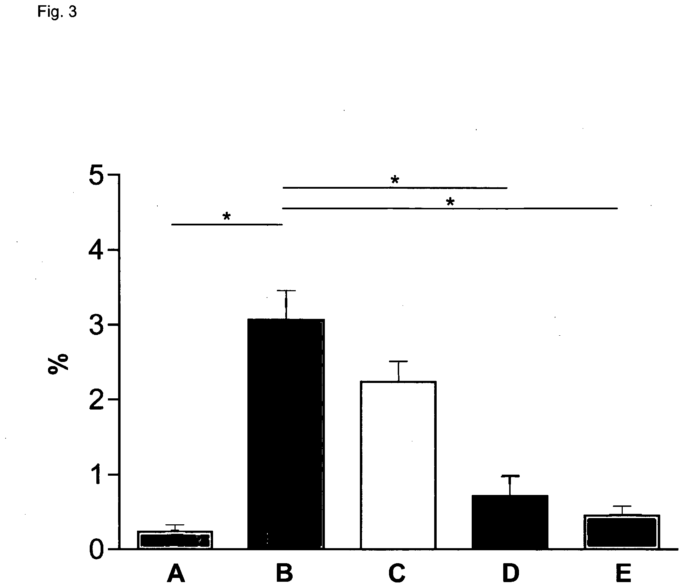

The results are depicted as bar diagrams in Fig. 3. The values (mean ± SEM) indicate the percentage of the stained surface in the evaluated histological samples (5 mice per group; 5 - 6 evaluated histological samples per mouse).

From this experiment it can be concluded that pre-treatment with 5-amino-2,3-dihydro-1,4-phthalazinedione sodium salt prevents cigarette smoke-induced 3-nitrotyrosine formation in the lung parenchyma in the ILU model.

This suggests that 5-amino-2,3-dihydro-1,4-phthalazinedione sodium salt has a protective effect against acute cigarette smoke-induced lung injury. Thus, these results can be regarded as predictive for a beneficial effect of 5-amino-2,3-dihydro-1,4-phthalazinedione and its pharmaceutically acceptable salts in the inhalatory prophylaxis and/or treatment of all inflammatory pulmonary diseases.

### Example 2:

The particle size distribution (FPM) and the mass median aerodynamic diameter (MMAD) of an aerosol generated from an aqueous solution containing 5-amino-2,3-dihydro-1,4-phthalazinedione sodium salt was analyzed.

Crystalline polymorph Form I of 5-amino-2,3-dihydro-1,4-phthalazinedione sodium salt was solved in physiological saline solution (double distilled water containing 0.9 % NaCl). The final concentration solution was 20 mg/ml 5-amino-2,3-dihydro-1,4-phthalazinedione sodium salt.

The experimental procedure was carried out pursuant to Ph. Eur. 2.9.44 (Preparations for Nebulisation: Characterisation). A cascade impactor (Next Generation Impactor (NGI), Copley Scientific Ltd., Nottingham, UK) was used to determine the respective percentage for each fraction of the nebulized particles. The cascade impactor was cooled to 4°C for the measurements

The NGI comprises the following features:
1) Designed and accepted by pharma and biotech industry for inhaler testing;
2) Meets and exceeds all European and US Pharmacopoeia specifications;
3) Particle size range between 0.24 - 11.7 µm (dependent on flow rate);
4) 7 stages, 5 with cut-offs between 0.54 - 6.12 µm at flow rates from 30 to 100 l/min;
5) Calibrated flow rate range from 30 - 100 l/min;
6) Additional calibration at 15 l/min for nebulizer applications;
7) Supplied with full stage mensuration report (system suitability);
8) Low inter-stage wall losses for good drug recovery (mass balance);
9) Electrically conductive and unaffected by static;

The NGI cascade impactor itself comprises three main parts:
a) The cup tray containing the eight collection cups used to collect the samples prior to analysis
b) The bottom frame used to support the cup tray
c) The lid containing the inter-stage passageways and the seal body which holds the nozzles in place.

The nebulization of this aqueous solution of 5-amino-2,3-dihydro-1,4-phthalazinedione sodium salt was carried out by means of a vibrating mesh nebulizer (M-neb-dose+, NEBU-TEC, Eisenfeld, Germany). The thus generated aerosol was delivered to the cascade impactor by means of a mouthpiece (SK-211, NEBU-TEC, Eisenfeld, Germany).

The nebulization chamber (yellow stamp, 250 µl liquid discharge) was filled. The puff profile configured at the breath simulator was chosen in such a way that a correct simulation of inhalation by means of the nebulizer was ensured. The corresponding time was recorded.

The quantification of nebulized 5-amino-2,3-dihydro-1,4-phthalazinedione sodium salt per cascade was carried out after extraction from the cascade pans (stage 1 - 8) by means of HPLC with reference to an external standard calibration curve (range 0 - 200 µg/ml; correlation coefficient: 0.9999).

Single values obtained for each cascade were added. No residual amounts of 5-amino-2,3-dihydro-1,4-phthalazinedione sodium salt could be found in the mouthpiece. MMAD, FPF and FPM were calculated from the obtained values:

| released dosage | 4.7 mg |
|---|---|
| MMAD | 3.1 µm |
| FPF | 74.3 % |
| FPF | 3.5 mg |
| FPM | 69.0 % |
| FPM | 3.3 mg |

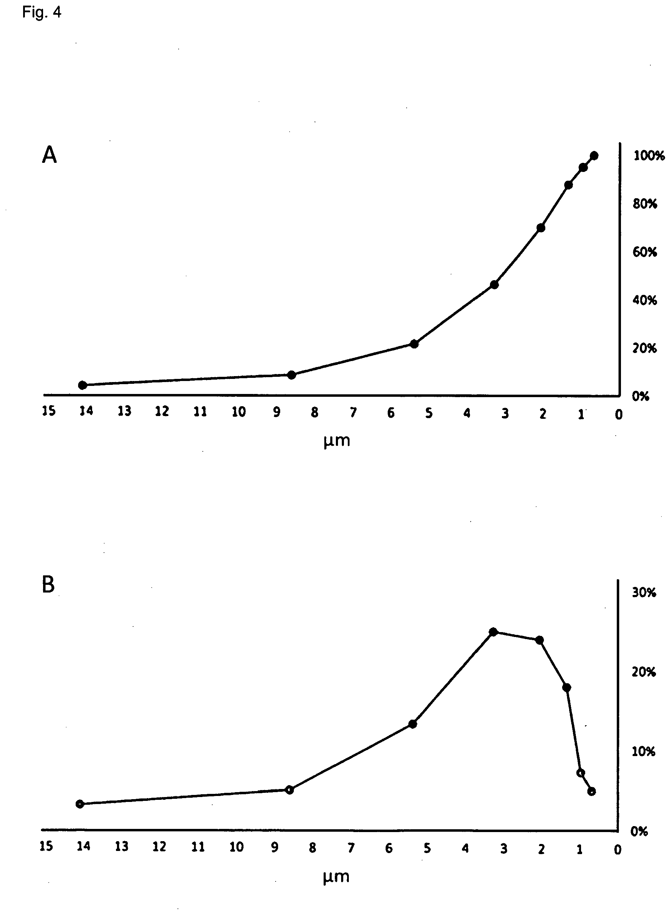

The cumulative particle size distribution is depicted in Fig. 4A, the relative particle size distribution in Fig. 4B.

The aerosol can be further characterized as follows:
- The released dosage at the mouthpiece of 4.7 mg corresponds to 95 % of the nominal value
- The time of aerosol release was 3 min (corresponds to 30 strokes / breaths)
- The ratio of particles with a MMAD < 1 µm to particles with a MMAD > 1 µm is 5 : 95.

### Example 3:

For comparison, the particle size distribution (FPF and FPM) and the mass median aerodynamic diameter (MMAD) of an aerosol generated from a physiological saline solution containing salbutamol sulfate was analyzed (not part of the invention). The procedure was the same as described in Example 2.

Salbutamol (albuterol) is a short-term bronchodilator. It consists of a racemic mixture of the pharmacologically active (*R*)-(-)-enantiomer that acts as a beta-2 receptor agonist and the metabolically active (*S*)-(+)-enantiomer. Activation of beta-2 receptors effects the conversion of ATP to cyclic AMP (cAMP) via adenylyl cyclase, triggering the intracellular signaling pathway which results in the inhibition of myosin phosphorylation and lowering the intracellular concentration of Ca²⁺. The increase in cAMP also inhibits inflammatory cells in the airways from releasing inflammatory mediators and cytokines. Salbutamol also increases the conductance of Ca²⁺and K⁺ channels, leading to a hyperpolarization and the relaxation of bronchial smooth muscles.

Salbutamol is one of the most widely used substances for acute asthma attacks. It is often administered via metered-dose inhalers. Therefore, a physiological saline solution of salbutamol sulfate was tested in order to determine the particle size distribution. The standard dosage (Albutol Sulfate) of 180 µg (90 µg/spray application, 2 spray applications per inhalation session) was dissolved in physiological saline.

Single values obtained for each cascade were added. No residual amounts of salbutamol sulfate could be found in the mouthpiece. MMAD, FPF and FPM were calculated from the obtained values:

| released dosage | 166 µg |
|---|---|
| MMAD | 3.57 µm |
| FPF | 73.3 % |
| FPF | 122 µg |
| FPM | 69.1 % |
| FPM | 115 µg |

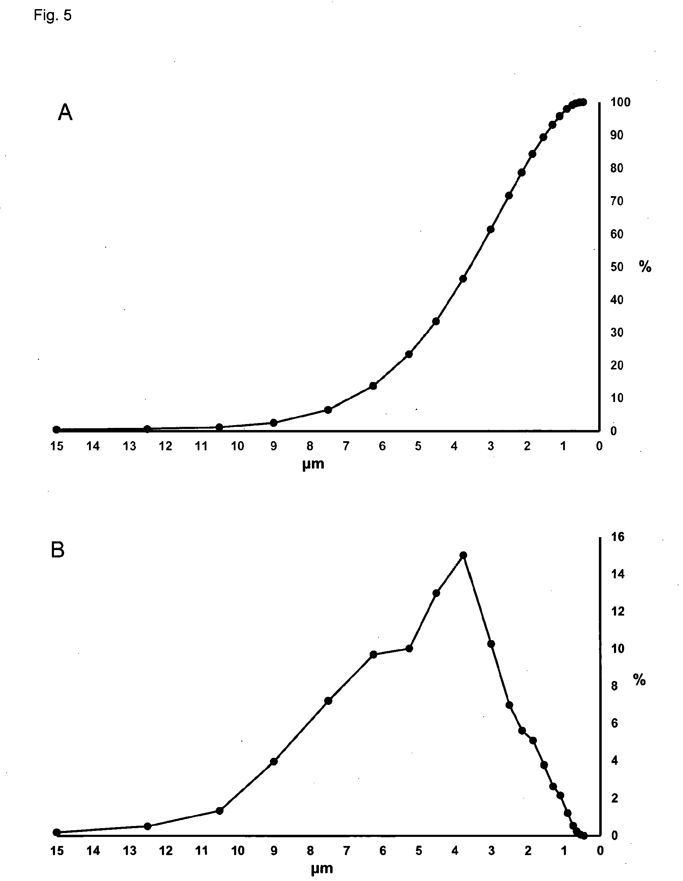

The cumulative particle size distribution is depicted in Fig. 5A, the relative particle size distribution in Fig. 5B.

The aerosol can be further characterized as follows:
- The released dosage at the mouthpiece of 166 µg corresponds to 92 % of the nominal value
- The time of aerosol release was 3 min (corresponds to 30 strokes / breaths)
- The ratio of particles with a MMAD < 1 µm to particles with a MMAD > 1 µm is 4 : 96.

In comparison, 5-amino-2,3-dihydro-1,4-phthalazinedione sodium salt shows a clearly smaller MMAD than salbutamol sulfate. FPF is slightly higher for 5-amino-2,3-dihydro-1,4-phthalazinedione sodium salt, while FPM is in a similar range.

### Example 4:

For comparison, the particle size distribution (FPF and FPM) and the mass median aerodynamic diameter (MMAD) of an aerosol generated from a physiological saline solution containing ipratropium bromide was analyzed (not part of the invention). The procedure was the same as described in Example 2.

Ipratropium bromide is a pharmaceutically active agent that is used in the treatment of COPD and cardiac arrythmias. It acts as a competitive antagonist at the muscarinic acetylcholine receptor, at the smooth muscles of the pulmonary vascular epithelium of the bronchi at the M₃ receptor. The M₃ receptor is G_{q}-coupled, leading to an increase in inositol triphosphate (IP₃) and subsequently to a Ca²⁺ release from intracellular calcium stores. Thus, a blockade of the M₃ receptor causes bronchodilation.

Ipratropium bromide is often administered via nebulizers and nasal sprays. Therefore, a physiological saline solution of ipratropium bromide was tested in order to determine the particle size distribution. The standard dosage (Atrovent) of 18 µg/spray application was dissolved in physiological saline.

Single values obtained for each cascade were added. No residual amounts of ipratropium bromide could be found in the mouthpiece. MMAD, FPF and FPM were calculated from the obtained values:

| released dosage | 15 µg |
|---|---|
| MMAD | 3.59 µm |
| FPF | 73.4 % |
| FPF | 11 µg |
| FPM | 69.4 % |
| FPM | 10 µg |

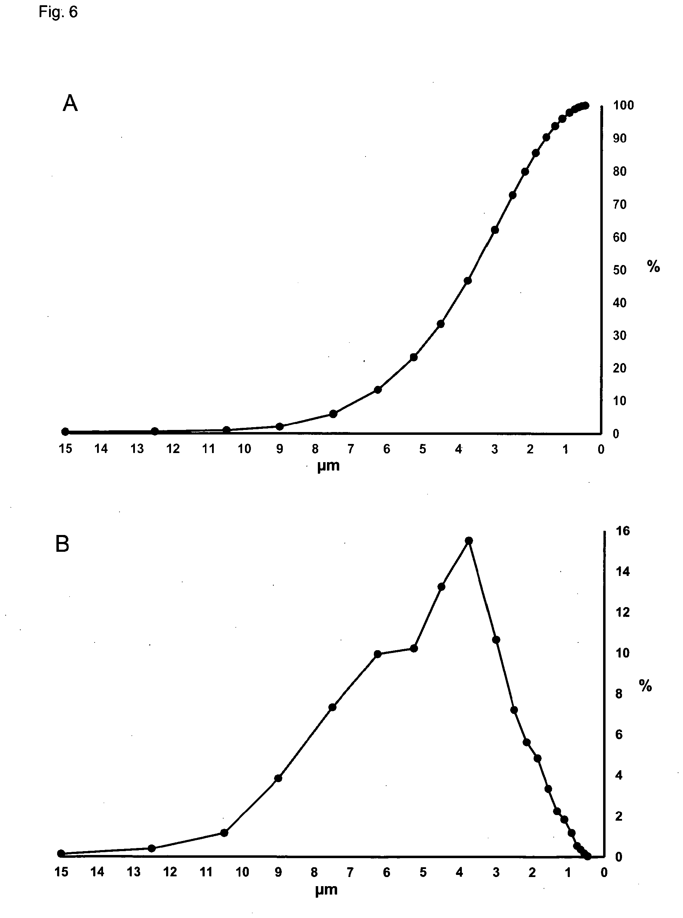

The cumulative particle size distribution is depicted in Fig. 6A, the relative particle size distribution in Fig. 6B.

The aerosol can be further characterized as follows:
- The released dosage at the mouthpiece of 15 µg corresponds to 86 % of the nominal value
- The time of aerosol release was 3 min (corresponds to 30 strokes / breaths)
- The ratio of particles with a MMAD < 1 µm to particles with a MMAD > 1 µm is 4 : 96.

In comparison, 5-amino-2,3-dihydro-1,4-phthalazinedione sodium salt shows a clearly smaller MMAD than ipratropium bromide. FPF is slightly higher for 5-amino-2,3-dihydro-1,4-phthalazinedione sodium salt, while FPM is slightly smaller.

### Figures

- Fig. 1:: A: Schematic drawing of the experimental setup of Example 1
   1 - cigarette smoke
   2 - ventilator
   3 - trachea
   4 - lung
   5 - heart
   6 - reservoir
   7 - aqueous solution of 5-amino-2,3-dihydro-1,4-phthalazinedione sodium salt
   8 - roller pump
B: Picture of the experimental setup of Example 1
- Fig. 2:: Immunohistochemical staining of representative samples from Example 1
Left panel: 200 x magnification
Right panel: 400 x magnification, enlarged detail from the left panel

A: room air
B: cigarette smoke + diluent (buffer solution)
C: cigarette smoke + 0.5 mM 5-amino-2,3-dihydro-1,4-phthalazinedione sodium salt
D: cigarette smoke + 1 mM 5-amino-2,3-dihydro-1,4-phthalazinedione sodium salt
E: cigarette smoke + 2 mM 5-amino-2,3-dihydro-1,4-phthalazinedione sodium salt
In the right panel the inflamed areas are highlighted.
- Fig. 3:: Statistical evaluation of the immunohistochemical staining of samples from Example 1. The percentage of stained surface area corresponds to the grade of inflammation (n = 5; mean ± SEM). Bars marked with an asterisk indicate a highly significant difference between two groups (p < 0.001).
A: room air
B: cigarette smoke + diluent (buffer solution)
C: cigarette smoke + 0.5 mM 5-amino-2,3-dihydro-1,4-phthalazinedione sodium salt
D: cigarette smoke + 1 mM 5-amino-2,3-dihydro-1,4-phthalazinedione sodium salt
E: cigarette smoke + 2 mM 5-amino-2,3-dihydro-1,4-phthalazinedione sodium salt
- Fig. 4:: Particle size distribution in µm of the aerosol generated in Example 2, determined as MMAD.
A: Cumulative MMAD
B: Relative MMAD
- Fig. 5:: Particle size distribution in µm of the aerosol generated in Example 3, determined as MMAD.
A: Cumulative MMAD
B: Relative MMAD
- Fig. 6:: Particle size distribution in µm of the aerosol generated in Example 4, determined as MMAD.
A: Cumulative MMAD
B: Relative MMAD

### List of abbreviations:

| | |
|---|---|
| ACES | 2-[(amino-2-oxoethyl)amino]ethanesulfonic acid |
| API | active pharmaceutical ingredient |
| ARDS | adult respiratory distress syndrome |
| BES | N,N-bis-(2-hydroxyethyl)-2-aminoethanesulfonic acid |
| BOS | bronchiolitis obliterans syndrome |
| BSA | bovine serum albumin |
| CBD | chronic beryllium disease |
| CFTR | cystic fibrosis transmembrane conductance regulator |
| CLAD | chronic lung allograft dysfunction |
| CLAD-BOS | chronic lung allograft dysfunction - bronchiolitis obliterans syndrome |
| COPD | chronic obstructive pulmonary disease |
| COX | cyclooxygenase |
| DAPI | 4',6-diamidino-2-phenylidone |
| DNA | deoxyribonucleic acid |
| DPI | dry powder inhaler |
| ECMO | extracorporeal membrane oxygenation |
| EEPS | 4-(2-hydroxyethyl)piperazine-1-propanesulfonic acid |
| ENaC | epithelial sodium channel |
| FEV1 | forced expiratory volume in the first second of expiration |
| FPF | fine particle fraction |
| FPM | fine particle mass |
| FVC | forced vital capacity |
| HAPE | high-altitude pulmonary edema |
| HEPES | N-2-hydroxyethylpiperazine-N'-2-ethanesulfonic acid |
| ICD-10 | International Statistical Classification of Diseases and Related Health Problems, 10th revision |
| IL | interleukin |
| IR | ischemia-reperfusion |
| LABA | long-acting beta-2 agonist |
| 5-LOX | 5-lipoxygenase |
| m² | square meter |
| MAD | mass aerodynamic diameter |
| MDI | metered-dose inhaler |
| MES | 2-(N-morpholino) ethanesulfonic acid |
| MMAD | median mass aerodynamic diameter |
| MOPS | 3-(N-morphino) propanesulfonic acid |
| µm | micrometer |
| NETs | neutrophil extracellular traps |
| NO | nitric oxide |
| NOX | NADPH oxidase |
| 3-NT | 3-nitrotyrosine |
| PBS | phosphate buffer saline |
| PDE | phosphodiesterase |
| PDGF | platelet-derived growth factor |
| PEEP | positive end-expiratory pressure |
| PEG | polyethylene glycol |
| PGD | primary graft dysfunction |
| PIPES | 4-piperazine-bis-ethanesulfonic acid |
| pMDI | pressurized metered-dose inhaler |
| PS | pulmonary sarcoidosis |
| RNS | reactive nitrogen species |
| ROS | reactive oxygen species |
| SEM | standard error of mean |
| TAPS | [(2-hydroxy-1,1-bis-(hydroxymethyl)ethyl)amino]-1-propanesulfonic acid |
| TBS | Tris wash buffer |
| TES | 2-[tris(hydroxymethyl)methyl]aminoethanesulfonic acid |
| TNF-alpha | tumor necrosis factor-alpha |

## Claims

1. 5-amino-2,3-dihydro-1,4-phthalazinedione or one of its pharmaceutically acceptable salts for use in the prophylaxis or treatment of inflammatory pulmonary diseases by inhalatory administration,
wherein a vibrating mesh nebulizer releases for the inhalatory administration an aerosol containing liquid droplets of 5-amino-2,3-dihydro-1,4-phthalazinedione or one of its pharmaceutically acceptable salts,
and the median mass aerodynamic diameter of the liquid droplets of the aerosol is between 3.0 µm and 3.3 µm,
and said aerosol is **characterized in that** the fine particle mass is at least 50% of the liquid droplets of the aerosol, wherein the fine particle mass indicates the percentage of the liquid droplets with a diameter in the range of 1.0 to 5.0 µm.

2. 5-amino-2,3-dihydro-1,4-phthalazinedione or one of its pharmaceutically acceptable salts for use according to claim 1, wherein said mesh nebulizer is a vibrating mesh nebulizer.

3. 5-amino-2,3-dihydro-1,4-phthalazinedione or one of its pharmaceutically acceptable salts for use according to any one of claims 1 or 2, wherein at least 50% of the liquid droplets of the aerosol have a mass aerodynamic diameter between 1.0 µm and 3.2 µm.

4. 5-amino-2,3-dihydro-1,4-phthalazinedione or one of its pharmaceutically acceptable salts for use according to any one of claims 1 to 3, wherein the pharmaceutically acceptable salt of 5-amino-2,3-dihydro-1,4-phthalazinedione is 5-amino-2,3-dihydro-1,4-phthalazinedione sodium salt.

5. 5-amino-2,3-dihydro-1,4-phthalazinedione or one of its pharmaceutically acceptable salts for use according to claim 4, wherein 5-amino-2,3-dihydro-1,4-phthalazinedione sodium salt is provided as one of crystalline anhydrate polymorph forms I, II or III **characterized by** crystallography values determined by means of x-ray powder diagrams:
d values: 13.5; 6.9; 5.2; 4.6; 3.9; 3.5; 3.4; 3.3; 3.1; 3.0 and/or
2-theta values: 6.5; 12.7; 16.9; 19.3; 22.8; 25.8; 26.6; 27.2; 28.7; 30.3 for form I, d values: 12.9; 7.9; 7.1; 6.5; 5.3; 4.0; 3.7; 3.6; 3.3; 3.2 and/or
2-theta values: 6.8; 11.2; 12.5; 13.7; 16.7; 22.4; 24.3; 24.9; 27.2; 27.8 for form II, and
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93 for form III.

6. 5-amino-2,3-dihydro-1,4-phthalazinedione or one of its pharmaceutically acceptable salts for use according to any one of claims 1 to 5, wherein the inflammatory pulmonary disease is selected from a group comprising inflammations of the lower airways due to a bacterial, viral, fungal or parasitic infection, chronic lower respiratory diseases, lung diseases due to an external agent, respiratory diseases principally affecting the interstitium, suppurative and/or necrotic conditions of lower respiratory tract, pleura diseases, postprocedural or related lower respiratory diseases, pulmonary diseases specific to the perinatal period, trauma and injuries of the lower respiratory tract and/or the thorax, and malignant neoplasms of the lower respiratory tract.

7. 5-amino-2,3-dihydro-1,4-phthalazinedione or one of its pharmaceutically acceptable salts for use according to any one of claims 1 to 6, wherein the inflammatory pulmonary disease is selected from a group comprising chronic obstructive pulmonary disease, asthma, sarcoidosis of the lung, cystic fibrosis, bronchiectasis, adult respiratory distress syndrome, pulmonary fibrosis, berylliosis, chronic lung allograft dysfunction, pulmonary edema, lung ischemia reperfusion injury and primary graft dysfunction after lung transplantation.

8. 5-amino-2,3-dihydro-1,4-phthalazinedione or one of its pharmaceutically acceptable salts for use in a therapy for the prophylaxis or treatment of inflammatory pulmonary diseases as aerosol,
wherein the median mass aerodynamic diameter of the liquid droplets of the aerosol is between 3.0 µm and 3.3 µm,
and said aerosol is **characterized in that** the fine particle mass is at least 50% of the liquid droplets of the aerosol, wherein the fine particle mass indicates the percentage of the liquid droplets with a diameter in the range of 1.0 to 5.0 µm.

9. An aerosol produced by a vibrating mesh nebulizer, containing 5-amino-2,3-dihydro-1,4-phthalazinedione or one of its pharmaceutically acceptable salts in the range of 0.01% per weight to 10 % per weight,
an aqueous solution in the range of 70 % per weight to 99.99 % per weight, and optionally at least one pharmaceutically acceptable excipient in the range of 0 % per weight to 20 % per weight,
wherein said percentages add up to 100 %,
the median mass aerodynamic diameter of the liquid droplets of the aerosol is between 3.0 µm and 3.3 µm
and said aerosol is **characterized in that** the fine particle mass is at least 50% of the liquid droplets of the aerosol, wherein the fine particle mass indicates the percentage of the liquid droplets with a diameter in the range of 1.0 to 5.0 µm.

10. A method for producing an aerosol as defined in claim 9, comprising the following steps:
a) filling 0.1 ml to 5 ml of an aqueous solution containing 5-amino-2,3-dihydro-1,4-phthalazinedione or one of its pharmaceutically acceptable salts and optionally at least one pharmaceutically acceptable excipient into the nebulization chamber of a mesh nebulizer,
b) starting vibration of the mesh of the mesh nebulizer at a frequency of 80 kHz to 200 kHz, and
c) discharging the generated aerosol at the side of the mesh of the mesh nebulizer opposite to the nebulization chamber.

11. The method according to claim 10,
**characterized in that** at least 90 % in weight of 5-amino-2,3-dihydro-1,4-phthalazinedione or one of its pharmaceutically acceptable salts contained in said aqueous solution are nebulized in the generated aerosol.

12. The method according to any one of claims 10 or 11,
**characterized in that** at least 80 % of the generated aerosol are produced during three minutes after starting nebulization in the mesh nebulizer.

13. A kit, comprising a vibrating mesh nebulizer and a pharmaceutically acceptable container with an aqueous solution containing 5-amino-2,3-dihydro-1,4-phthalazinedione or one of its pharmaceutically acceptable salts and optionally at least one pharmaceutically acceptable excipient,
wherein said vibrating mesh nebulizer is able to produce an aerosol from said aqueous solution that is **characterized in that** the median mass aerodynamic diameter of the liquid droplets of the aerosol is between 3.0 µm and 3.3 µm, and
**in that** the fine particle mass is at least 50% of the liquid droplets of the aerosol, wherein the fine particle mass indicates the percentage of the liquid droplets with a diameter in the range of 1.0 to 5.0 µm.

14. A kit, comprising a vibrating mesh nebulizer, a first pharmaceutically acceptable container with water for injection or physiological saline solution and a second pharmaceutically acceptable container with a solid form of 5-amino-2,3-dihydro-1,4-phthalazinedione or one of its pharmaceutically acceptable salts,
wherein optionally at least one pharmaceutically acceptable excipient is contained in the first pharmaceutically acceptable container and/or the second pharmaceutically acceptable container, and
said vibrating mesh nebulizer is able to produce an aerosol from a solution resulting from the mixing of the content of the first container and the second container, and said aerosol is **characterized in that** the median mass aerodynamic diameter of the liquid droplets of the aerosol is between 3.0 µm and 3.3 µm, and
**in that** the fine particle mass is at least 50% of the liquid droplets of the aerosol, wherein the fine particle mass indicates the percentage of the liquid droplets with a diameter in the range of 1.0 to 5.0 µm.

15. A kit as defined in any one of claims 13 or 14, additionally comprising a mouthpiece for inhalation fitting to said mesh nebulizer.

## Patentansprüche

1. 5-Amino-2,3-dihydro-1,4-phthalazindion oder eines seiner pharmazeutisch verträglichen Salze zur Verwendung in der Prophylaxe oder Behandlung entzündlicher pulmonaler Erkrankungen durch inhalative Verabreichung,
wobei ein Schwingmembran-Vernebler für die inhalative Verabreichung ein Aerosol freisetzt, das Flüssigkeitströpfchen von 5-Amino-2,3-dihydro-1,4-phthalazindion oder einem seiner pharmazeutisch verträglichen Salze enthält,
und der mediane massenbezogene aerodynamische Durchmesser zwischen 3,0 µm und 3,3 µm liegt,
und besagtes Aerosol **dadurch gekennzeichnet ist, dass** die feine Partikelmasse mindestens 50% des Aerosols ausmacht, wobei die feine Partikelmasse den Prozentsatz der Flüssigkeitströpfchen mit einem Durchmesser im Bereich von 1,0 bis 5,0 µm angibt.

2. 5-Amino-2,3-dihydro-1,4-phthalazindion oder eines seiner pharmazeutisch verträglichen Salze zur Verwendung gemäß Anspruch 1, wobei der Membranvernebler ein Schwingmembran-Vernebler ist.

3. 5-Amino-2,3-dihydro-1,4-phthalazindion oder eines seiner pharmazeutisch verträglichen Salze zur Verwendung gemäß einem der Ansprüche 1 oder 2, wobei mindestens 50% der Flüssigkeitströpfchen des Aerosols einen massenbezogenen aerodynamischen Durchmesser zwischen 1,0 µm und 3,2 µm aufweisen.

4. 5-Amino-2,3-dihydro-1,4-phthalazindion oder eines seiner pharmazeutisch verträglichen Salze zur Verwendung gemäß einem der Ansprüche 1 bis 3, wobei das pharmazeutisch verträgliche Salz von 5-Amino-2,3-dihydro-1,4-phthalazindion 5-Amino-2,3-dihydro-1,4-phthalazindion Natriumsalz ist.

5. 5-Amino-2,3-dihydro-1,4-phthalazindion oder eines seiner pharmazeutisch verträglichen Salze zur Verwendung gemäß Anspruch 4, wobei 5-Amino-2,3-dihydro-1,4-phthalazindion Natriumsalz als eine der kristallinen Anhydrat-Polymorphformen I, II oder III zur Verfügung gestellt wird, die durch die durch Röntgenpulverdiffraktometrie bestimmte kristallographische Werte charakterisiert sind:
D-Werte: 13,5; 6,9; 5,2; 4,6; 3,9; 3,5; 3,4; 3,3; 3,1; 3,0 und/oder
2-Theta-Werte: 6,5; 12,7; 16,9; 19,3; 22,8; 25,8; 26,6; 27,2; 28,7; 30,3 für Form I,
D-Werte: 12,9; 7,9; 7,1; 6,5; 5,3; 4,0; 3,7; 3,6; 3,3; 3,2 und/oder
2-Theta-Werte: 6,8; 11,2; 12,5; 13,7; 16,7; 22,4; 24,3; 24,9; 27,2; 27,8 für Form II, und
D-Werte: 13,131; 7,987; 7,186; 6,566; 6,512; 5,372; 3,994; 3,662; 3,406; 3,288; 3,283; 3,222; 3,215; 3,127; 2,889 und/oder
2-Theta-Werte: 6,73; 11,07; 12,31; 13,48; 13,59; 16,49; 22,24; 24,29; 26,14; 27,10; 27,14; 27,67; 27,72; 28,52; 30,93 für Form III.

6. 5-Amino-2,3-dihydro-1,4-phthalazindion oder eines seiner pharmazeutisch verträglichen Salze zur Verwendung gemäß einem der Ansprüche 1 bis 5, wobei die entzündliche pulmonale Erkrankung aus einer Gruppe ausgewählt wird, die Entzündungen der unteren Atemwege aufgrund einer bakteriellen, viralen, oder fungalen Infektion oder durch Parasiten, chronische Erkrankungen der unteren Atemwege, Lungenkrankheiten durch exogene Substanzen, Erkrankungen der Atmungsorgane, die das Interstitium betreffen, purulente und/oder nekrotisierende Zustände der unteren Atemwege, Erkrankungen der Pleura, postoperative oder verwandte Erkrankungen der unteren Atemwege, für den perinatalen Zeitraum spezifische Lungenerkrankungen, Trauma und Verletzungen der unteren Atemwege und/oder des Thorax sowie maligne Neoplasmen der unteren Atemwege umfasst.

7. 5-Amino-2,3-dihydro-1,4-phthalazindion oder eines seiner pharmazeutisch verträglichen Salze zur Verwendung gemäß einem der Ansprüche 1 bis 6, wobei die entzündliche pulmonale Erkrankung aus einer Gruppe ausgewählt wird, die chronische obstruktive Lungenkrankheit, Asthma, Sarkoidose der Lunge, zystische Fibrose, Bronchiektasie, akutes Lungenversagen, Lungenfibrose, Berylliose, chronische Funktionsstörungen bei Lungentransplantaten, Lungenödem, Ischämie-Reperfusionsverletzungen der Lunge und primäre Transplantatdysfunktion nach einer Lungentransplantation umfasst.

8. 5-Amino-2,3-dihydro-1,4-phthalazindion oder eines seiner pharmazeutisch verträglichen Salze zur Verwendung in einer Therapie zur Prophylaxe oder Behandlung entzündlicher pulmonaler Erkrankungen als Aerosol,
wobei der mediane massenbezogene aerodynamische Durchmesser zwischen 3,0 µm und 3,3 µm liegt,
und besagtes Aerosol **dadurch gekennzeichnet ist, dass** die feine Partikelmasse mindestens 50% des Aerosols ausmacht, wobei die feine Partikelmasse den Prozentsatz der Flüssigkeitströpfchen mit einem Durchmesser im Bereich von 1,0 bis 5,0 µm angibt.

9. Ein durch einen Schwingmembran-Vernebler hergestelltes Aerosol, das 5-Amino-2,3-dihydro-1,4-phthalazindion oder eines seiner pharmazeutisch verträglichen Salze in einem Bereich von 0,01 Gew.-% bis 10 Gew.-%,
eine wässrige Lösung in einem Bereich von 70 Gew.-% bis 99,99 Gew.-%, und optional mindestens einen pharmazeutisch verträglichen Hilfsstoff in einem Bereich von 0 Gew.-% bis 20 Gew.-% enthält,
wobei sich besagte Prozentsätze zu 100 % summieren,
der mediane massenbezogene aerodynamische Durchmesser zwischen 3,0 µm und 3,3 µm liegt,
und besagtes Aerosol **dadurch gekennzeichnet ist, dass** die feine Partikelmasse mindestens 50% des Aerosols ausmacht, wobei die feine Partikelmasse den Prozentsatz der Flüssigkeitströpfchen mit einem Durchmesser im Bereich von 1,0 bis 5,0 µm angibt.

10. Ein Verfahren zur Herstellung eines wie in Anspruch 9 definierten Aerosols, das die folgenden Schritte umfasst:
a) Füllen von 0,1 ml bis 5 ml einer wässrigen Lösung, die 5-Amino-2,3-dihydro-1,4-phthalazindion oder eines seiner pharmazeutisch verträglichen Salze und mindestens einen pharmazeutisch verträglichen Hilfsstoff enthält, in die Vernebelungskammer eines Membranverneblers,
b) Starten der Schwingung der Membran des Membranverneblers mit einer Frequenz von 80 kHz bis 200 kHz, und
c) Abgeben des erzeugten Aerosols auf der der Vernebelungskammer abgewandten Seite der Membran des Membranverneblers.

11. Das Verfahren gemäß Anspruch 10,
dadurch charakterisiert, dass mindestens 90 Gew.-% des in besagter wässriger Lösung enthaltenen 5-Amino-2,3-dihydro-1,4-phthalazindions oder eines seiner pharmazeutisch verträglichen Salze in dem erzeugten Aerosol vernebelt werden.

12. Das Verfahren gemäß einem der Ansprüche 10 oder 11,
dadurch charakterisiert, dass mindestens 80 % des erzeugten Aerosols innerhalb von drei Minuten nach dem Start der Vernebelung im Membranvernebler erzeugt werden.

13. Ein Kit, der einen Schwingmembran-Vernebler und ein pharmazeutisch zulässiges Behältnis mit einer wässrigen Lösung, 5-Amino-2,3-dihydro-1,4-phthalazindion oder einem seiner pharmazeutisch verträglichen Salze und optional mindestens einen pharmazeutisch verträglichen Hilfsstoff enthält, umfasst,
wobei der besagte Schwingmembran-Vernebler in der Lage ist, ein Aerosol der besagten wässrigen Lösung zu erzeugen, das dadurch charakterisiert ist, dass der mediane massenbezogene aerodynamische Durchmesser zwischen 3,0 µm und 3,3 µm liegt und die feine Partikelmasse mindestens 50% des Aerosols ausmacht, wobei die feine Partikelmasse den Prozentsatz der Flüssigkeitströpfchen mit einem Durchmesser im Bereich von 1,0 bis 5,0 µm angibt.

14. Ein Kit, das einen Schwingmembran-Vernebler, ein erstes pharmazeutisch zulässiges Behältnis mit Wasser für Injektionszwecke oder physiologischer Kochsalzlösung und ein zweites pharmazeutisch zulässiges Behältnis mit einer Festform von 5-Amino-2,3-dihydro-1,4-phthalazindion oder einem seiner pharmazeutisch verträglichen Salze umfasst,
wobei optional mindestens ein pharmazeutisch verträglicher Hilfsstoff im ersten pharmazeutisch zulässigen Behältnis und/oder dem zweiten pharmazeutisch zulässigen Behältnis enthalten ist, und
der besagte Schwingmembran-Vernebler in der Lage ist, ein Aerosol von einer Lösung zu erzeugen, die aus der Mischung des Inhalts des ersten Behältnisses und des zweiten Behältnisses hergestellt wird, und
das besagte Aerosol dadurch charakterisiert ist, dass der mediane massenbezogene aerodynamische Durchmesser zwischen 3,0 µm und 3,3 µm liegt und die feine Partikelmasse mindestens 50% des Aerosols ausmacht, wobei die feine Partikelmasse den Prozentsatz der Flüssigkeitströpfchen mit einem Durchmesser im Bereich von 1,0 bis 5,0 µm angibt.

15. Ein wie in einem der Ansprüche 13 oder 14 definierter Kit, der zusätzlich ein Mundstück zur Inhalation umfasst, dass in den besagten Membranvernebler passt.

## Revendications

1. 5-amino-2,3-dihydro-1,4-phthalazinedione ou l'un de ses sels pharmaceutiquement acceptables pour utilisation dans la prophylaxie ou le traitement des maladies pulmonaires inflammatoires par administration par inhalation,
dans ce qu'un nébuliseur à maille vibrante libère pour l'administration par inhalation un aérosol contenant des gouttelettes liquides de 5-amino-2,3-dihydro-1,4-phthalazinedione ou de l'un de ses sels pharmaceutiquement acceptables,
et le diamètre aérodynamique massique médian des gouttelettes liquides de l'aérosol est compris entre 3,0 µm et 3,3 µm,
et ledit aérosol est **caractérisé par le fait que** la masse des particules fines représente au moins 50 % des gouttelettes liquides de l'aérosol, la masse des particules fines indiquant le pourcentage des gouttelettes liquides dont le diamètre est compris entre 1,0 et 5,0 µm .

2. 5-amino-2,3-dihydro-1,4-phthalazinedione ou l'un de ses sels pharmaceutiquement acceptables pour l'utilisation selon la revendication 1, dans laquelle ledit nébuliseur à maille est un nébuliseur à maille vibrante.

3. 5-amino-2,3-dihydro-1,4-phtalazinedione ou l'un de ses sels pharmaceutiquement acceptables pour l'utilisation selon l'une quelconque des revendications 1 ou 2, dans lequel au moins 50 % des gouttelettes liquides de l'aérosol ont un diamètre aérodynamique massique compris entre 1,0 µm et 3,2 µm.

4. 5-amino-2,3-dihydro-1,4-phthalazinedione ou l'un de ses sels pharmaceutiquement acceptables pour l'utilisation selon l'une des revendications 1 à 3, dans laquelle le sel pharmaceutiquement acceptable de 5-amino-2,3-dihydro-1,4-phthalazinedione est le sel de sodium de 5-amino-2,3-dihydro-1,4-phthalazinedione.

5. 5-amino-2,3-dihydro-1,4-phthalazinedione ou l'un de ses sels pharmaceutiquement acceptables pour l'utilisation selon la revendication 4, dans lequel le sel de sodium de 5-amino-2,3-dihydro-1,4-phthalazinedione est fourni sous l'une des Formes polymorphes anhydriques cristallines I, Il ou III **caractérisées par** des valeurs cristallographiques déterminées au moyen de diagrammes de poudres aux rayons X :
valeurs d: 13,5; 6,9; 5,2; 4,6; 3,9; 3,5; 3,4; 3,3; 3,1; 3,0 et/ou
valeurs 2-théta: 6,5; 12,7; 16,9; 19,3; 22,8; 25,8; 26,6; 27,2; 28,7; 30,3 pour la Forme I,
valeurs d: 12,9; 7,9; 7,1; 6,5; 5,3; 4,0; 3,7; 3,6; 3,3; 3,2 et/ou
valeurs 2-théta: 6,8; 11,2; 12,5; 13,7; 16,7; 22,4; 24,3; 24,9; 27,2; 27,8 pour la Forme II, et
valeurs d: 13,131; 7,987; 7,186; 6,566; 6,512; 5,372; 3,994; 3,662; 3,406; 3,288; 3,283; 3,222; 3,215; 3,127; 2,889 et/ou
valeurs 2-théta: 6,73; 11,07; 12,31; 13,48; 13,59; 16,49; 22,24; 24,29; 26,14; 27,10; 27,14; 27,67; 27,72; 28,52; 30,93 pour la Forme III.

6. 5-amino-2,3-dihydro-1,4-phtalazinedione ou l'un de ses sels pharmaceutiquement acceptables pour l'utilisation selon l'une quelconque des revendications 1 à 5, dans laquelle la maladie pulmonaire inflammatoire est choisie dans un groupe comprenant les inflammations des voies respiratoires inférieures dues à une infection bactérienne, virale, fongique ou parasitaire, les maladies chroniques des voies respiratoires inférieures, les maladies pulmonaires dues à un agent externe, les maladies respiratoires affectant principalement l'interstitium, les états suppuratifs et/ou nécrotiques des voies respiratoires inférieures, les maladies de la plèvre, les maladies respiratoires inférieures postprocedurales ou connexes, les maladies pulmonaires spécifiques à la période périnatale, les traumatismes et les blessures des voies respiratoires inférieures et/ou du thorax, et les néoplasmes malins des voies respiratoires inférieures.

7. 5-amino-2,3-dihydro-1,4-phtalazinedione ou l'un de ses sels pharmaceutiquement acceptables pour l'utilisation selon l'une quelconque des revendications 1 à 6, dans laquelle la maladie pulmonaire inflammatoire est choisie dans un groupe comprenant maladie pulmonaire obstructive chronique, asthme, sarcoïdose pulmonaire, mucoviscidose, bronchectasie, syndrome de détresse respiratoire de l'adulte, fibrose pulmonaire, bérylliose, dysfonctionnement chronique de l'allogreffe pulmonaire, oedème pulmonaire, lésion d'ischémie reperfusion pulmonaire et dysfonctionnement primaire du greffon après transplantation pulmonaire.

8. 5-amino-2,3-dihydro-1,4-phtalazinedione ou l'un de ses sels pharmaceutiquement acceptables pour utilisation dans une thérapie pour la prophylaxie ou le traitement des maladies pulmonaires inflammatoires sous forme d'aérosol,
dans lequel le diamètre aérodynamique massique médian des gouttelettes liquides de l'aérosol est compris entre 3,0 µm et 3,3 µm,
et ledit aérosol est **caractérisé par le fait que** la masse des particules fines représente au moins 50 % des gouttelettes liquides de l'aérosol, la masse des particules fines indiquant le pourcentage des gouttelettes liquides dont le diamètre est compris entre 1,0 et 5,0 µm.

9. Aérosol produit par un nébuliseur à maille vibrante, contenant 5-amino-2,3-dihydro-1,4-phtalazinedione ou l'un de ses sels pharmaceutiquement acceptables comprise entre 0,01 % en poids et 10 % en poids,
une solution aqueuse comprise entre 70 % en poids et 99,99 % en poids, et optionnellement, au moins un excipient pharmaceutiquement acceptable compris entre 0 % en poids et 20 % en poids,
dans lequel la somme des pourcentages est égale à 100 %,
le diamètre aérodynamique massique médian des gouttelettes liquides de l'aérosol est compris entre 3,0 µm et 3,3 µm,
et ledit aérosol est **caractérisé par le fait que** la masse des particules fines représente au moins 50 % des gouttelettes liquides de l'aérosol, la masse des particules fines indiquant le pourcentage des gouttelettes liquides dont le diamètre est compris entre 1,0 et 5,0 µm.

10. Méthode de production d'un aérosol telle que définie dans la revendication 9, comprenant les étapes suivantes:
introduire 0,1 ml à 5 ml d'une solution aqueuse contenant de la 5-amino-2,3-dihydro-1,4-phtalazinedione ou l'un de ses sels pharmaceutiquement acceptables et
optionnellement au moins un excipient pharmaceutiquement acceptable dans la chambre de nébulisation d'un nébuliseur à maille,
faire vibrer la maille du nébuliseur à une fréquence comprise entre 80 kHz et 200 kHz, et
l'évacuation de l'aérosol généré du côté de la maille du nébuliseur à maille opposé à la chambre de nébulisation.

11. La méthode selon la revendication 10,
**caractérisée par le fait qu'**au moins 90 % en poids de la 5-amino-2,3-dihydro-1,4-phthalazinedione ou de l'un de ses sels pharmaceutiquement acceptables contenus dans ladite solution aqueuse sont nébulisés dans l'aérosol généré.

12. La méthode selon l'une des revendications 10 ou 11,
**caractérisée par le fait qu'**au moins 80 % de l'aérosol généré est produit pendant les trois minutes qui suivent le début de la nébulisation dans le nébuliseur à mailles.

13. Kit comprenant un nébuliseur à maille vibrante et un récipient pharmaceutiquement acceptable contenant une solution aqueuse de 5-amino-2,3-dihydro-1,4-phtalazinedione ou l'un de ses sels pharmaceutiquement acceptables et optionnellement au moins un excipient pharmaceutiquement acceptable,
dans lequel ledit nébuliseur à maille vibrante est capable de produire un aérosol à partir de ladite solution aqueuse, **caractérisé par le fait que** le diamètre aérodynamique massique médian des gouttelettes liquides de l'aérosol est compris entre 3,0 µm et 3,3 µm, et
la masse de particules fines représente au moins 50 % des gouttelettes de liquide de l'aérosol, la masse de particules fines indiquant le pourcentage de gouttelettes de liquide dont le diamètre est compris entre 1,0 et 5,0 µm .

14. Kit comprenant un nébuliseur à maille vibrante, un premier récipient pharmaceutiquement acceptable contenant de l'eau pour injection ou une solution saline physiologique et un second récipient pharmaceutiquement acceptable contenant une forme solide de 5-amino-2,3-dihydro-1,4-phthalazinedione ou l'un de ses sels pharmaceutiquement acceptables,
dans lequel, optionnellement, au moins un excipient pharmaceutiquement acceptable est contenu dans le premier récipient pharmaceutiquement acceptable et/ou dans le second récipient pharmaceutiquement acceptable, et
ledit nébuliseur à maille vibrante est capable de produire un aérosol à partir d'une solution résultant du mélange du contenu du premier récipient et du second récipient, et ledit aérosol est **caractérisé en ce que** le diamètre aérodynamique massique médian des gouttelettes liquides de l'aérosol est compris entre 3,0 µm et 3,3 µm, et **en ce que** la masse de particules fines représente au moins 50 % des gouttelettes de liquide de l'aérosol, la masse de particules fines indiquant le pourcentage de gouttelettes de liquide dont le diamètre est compris entre 1,0 et 5,0 µm .

15. Kit tel que défini dans l'une des revendications 13 ou 14, comprenant en outre un embout buccal pour inhalation s'adaptant audit nébuliseur à mailles.
